# EUROPEAN PATENT SPECIFICATION

(11) **EP 3 522 906 B1**
(45) Date of publication and mention of the grant of the patent: **23.03.2022**
(21) Application number: 17859077.4
(22) Date of filing: 04.10.2017
(51) Int. Cl.: A61K 38/00, A61K 38/10, A61K 38/39, A61P 9/10

(54) **COMPOUNDS AND METHODS FOR ACTIVATING TIE2 SIGNALING**
VERBINDUNGEN UND VERFAHREN ZUR AKTIVIERUNG VON TIE2-SIGNALISIERUNG
COMPOSÉS ET PROCÉDÉS D'ACTIVATION DE LA SIGNALISATION TIE2

(30) Priority: 04.10.2016 US 201662403786 P
(43) Date of publication of application: 14.08.2019
(73) Proprietor: Asclepix Therapeutics, Inc., Baltimore, MD 21211 (US); The Johns Hopkins University, Baltimore, MD 21218 (US)
(72) Inventor: PANDEY, Niranjan, B., Baltimore, MD 21211 (US); MIRANDO, Adam, Baltimore MD 21218 (US); POPEL, Aleksander, S., Baltimore MD 21218 (US); GREEN, Jordan, J., Baltimore MD 21218 (US)
(74) Representative: Grund, Martin
(86) International application number: PCT/US2017/055055
(87) International publication number: WO 2018/067646

(56) References cited:
- US-A1- 2014 045 757
- US-A1- 2016 122 390
- ESAK LEE ET AL: "Inhibition of breast cancer growth and metastasis by a biomimetic peptide", SCIENTIFIC REPORTS, vol. 4, 20 November 2014 (2014-11-20), page 7139, XP055296374, DOI: 10.1038/srep07139

## Description

This application claims priority to U.S. Provisional Patent Application No. 62/403,786, filed October 4, 2016.

### BACKGROUND

The Tie2 receptor tyrosine kinase signaling pathway, and its ligands Angiopoietin1 (Ang1) and Angiopoietin2 (Ang2), regulate vascular permeability. Vascular permeability is compromised in patients with macular edema including patients with retinal vein occlusion (RVO), diabetic macular edema (DME), wet age-related macular degeneration (wet AMD), and background diabetic retinopathy (DR), as well as many other conditions. Tie2 may also regulate lymphatic vessel integrity especially during inflammation.

Ang1 binds Tie2 and stimulates phosphorylation and downstream signaling stabilizing blood vessels. Ang2 competes with Ang1 for Tie2 binding reducing the phosphorylation of Tie2, and thus it acts as an endogenous Tie2 antagonist. Ischemic or hypoxic retina produces high levels of Ang2, and Ang2 levels, like that of VEGF levels, are increased in the eyes of DME patients. Ang2 increases the responsiveness of retinal vessels to VEGF and promotes vascular leakage and neovascularization.

Ang2 may also act as a weak agonist of Tie2 especially when Ang1 levels are low. Specifically, exogenous Ang2 activates Tie2 and the promigratory, prosurvival PI3K/Akt pathway in endothelial cells (ECs) but with less potency and lower affinity than exogenous Ang1. ECs produce Ang2 but not Ang1. This endogenous Ang2 maintains Tie2, phosphatidylinositol 3-kinase, and Akt activities, and it promotes EC survival, migration, and tube formation.

Restoration of Tie2 activation could provide benefit in conditions associated with edema and vascular integrity, including macular edema, DME, and other conditions.

Lee *et al.* disclose the administration of 10 or 20 mg/kg non-encapsulated SP2043 per mouse. US 2016/122390 is directed to a peptide comprising an amino acid sequence which is at least 85 % identical to LRRFSTAPFAFIDINDVINF. In the examples of US 2016/122390, both non-encapsulated and encapsulated SP2043 is used in the treatment of ocular angiogenesis or diabetic retinopathy and also breast cancer.

### SUMMARY OF THE INVENTION

The invention is defined by the appended claims. In one aspect, the invention provides compositions for use in the treatment of Tie2-related vascular permeability, by administering one or more collagen IV-derived biomimetic peptides, wherein the peptide comprises an amino acid sequence with the formula LRRFSTXPXXXXDINDVXNF (SEQ ID NO:4) or LRRFSTXPXXXXNINNVXNF (SEQ ID NO:2), wherein X is a standard amino acid or non-genetically encoded amino acid, wherein the formulation comprises from 100 µg to about 1000 µg of the peptide agent, wherein the formulation does not involve encapsulation into particles, for use in the treatment of diabetic macular edema, retinal vein occlusion, wet age-related macular degeneration (wet AMD), or diabetic retinopathy, by intravitreal injection wherein the frequency of administration is no more frequent than about once every three months . Such peptides can promote the Tie2 agonist activities of Angiopoietin 2 (Ang2), thereby stabilizing vasculature and/or lymphatic vessels. In some embodiments, the frequency of administration is once every four or once every six months. In some embodiments, the dose of the peptide is from about 200 µg to about 800 µg or from about 200 µg to about 800 µg. In some embodiments, the X at position 7 of SEQ ID NO: 4 or SEQ ID NO: 2 is M, A, or G; X at position 9 of SEQ ID NO: 4 or SEQ ID NO: 2 is F, A, Y, or G; X at position 10 of SEQ ID NO: 4 or SEQ ID NO: 2 is M, A, G, D-Alanine (dA), or norleucine (Nle); X at position 11 of SEQ ID NO: 4 or SEQ ID NO: 2 is F, A, Y, G, or 4-chlorophenylalanine (4-ClPhe); X at position 12 and position 18 of SEQ ID NO: 4 or SEQ ID NO: 2 are independently selected from 2-Aminobutyric acid (Abu), G, S, A, V, T, I, L, or Allylglycine (AllylGly). In some embodiments, the peptide has the amino acid sequence LRRFSTAPFAFIDINDVINF (SEQ ID NO: 3). The biomimetic peptide is delivered for treatment of conditions such as diabetic macular edema, retinal vein occlusion, wet AMD, or diabetic retinopathy. In some embodiments, the condition is refractory or only partially-responsive to VEGF blockade therapy or kinase inhibitor therapy. For example, the biomimetic peptide is administered after unsuccessful VEGF blockade therapy, that is, where significant reductions in angiogenesis, lymphangiogenesis, and/or edema were not observed. In some embodiments, the peptide is administered as an alternative to, or in combination with, VEGF blockade therapy. By activating Tie2 signaling, the biomimetic peptides or peptide agents provide therapeutic benefits that may not be observed with VEGF-blockage therapy, or with VEGF blockade therapy alone.

Collagen IV-derived biomimetic peptides are derived from the α5 fibril of type IV collagen. The peptides may target α5β1 and αVβ3 integrins, and may inhibit signaling through multiple receptors, including vascular endothelial growth factor receptor (VEGFR), hepatocyte growth factor receptor (HGFR), insulin-like growth factor receptor (IGFR), and epidermal growth factor receptor (EGFR). As disclosed herein, collagen IV-derived biomimetic peptides further promote Tie2 agonist activities of Angiopoietin 2, thereby stabilizing vasculature and/or lymphatic vessels.

The biomimetic peptide or peptide agent is formulated for local delivery to affected tissues.

The invention provides compositions for use in preventing or treating a condition involving Tie-2-related vascular permeability or lymphatic vessel integrity in a patient. The compositions for use comprise administering the collagen IV-derived biomimetic peptide, , to the patient in an amount effective to reduce Tie2-dependent vascular permeability or lymphatic vessel integrity. Restoration of Tie2 activation provides therapeutic benefit in conditions associated with edema or vascular permeability, including macular edema, diabetic macular edema (DME), and other conditions. Tie2-related conditions include diabetic macular edema, retinal vein occlusion, wet AMD, and background diabetic retinopathy,

### BRIEF DESCRIPTION OF THE DRAWINGS

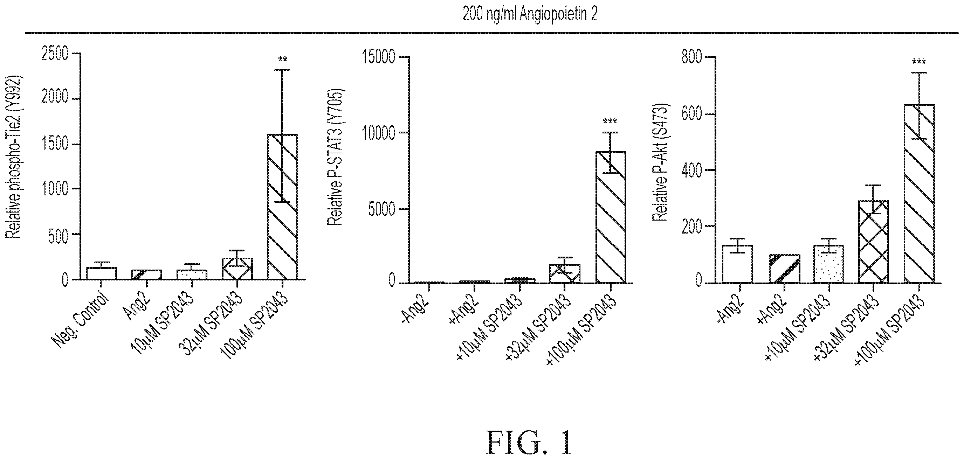

**FIG.** 1 shows that AXT107 (identified in FIG. 1 as SP2043) promotes the agonist activity of Ang2 to activate the Tie2 signaling pathway.

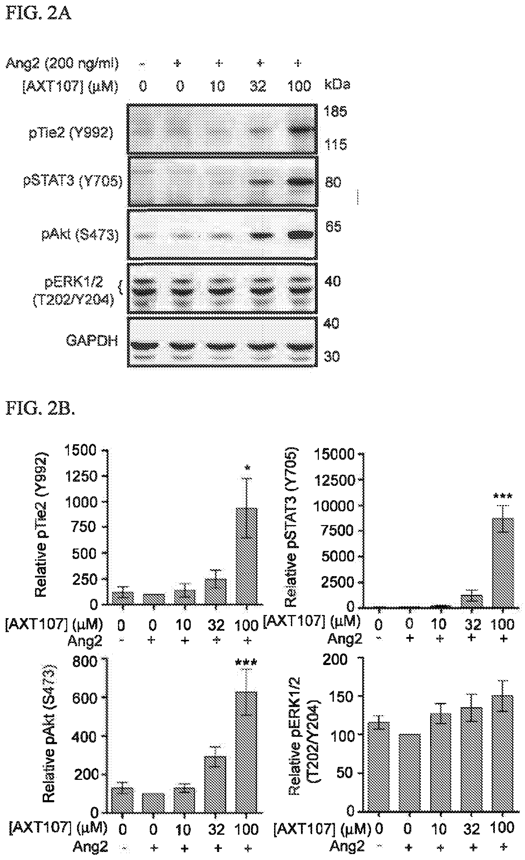

FIG. 2A shows western blots of microvascular endothelial cell (MEC) lysates treated with Ang2 and AXT107 showing phosphorylation of Tie2 and the downstream effectors STAT3, Akt, and Erk1/2, with GAPDH as a loading control. FIG. 2B shows graphs illustrating densitometric analyses of the western blots disclosed in FIG. 2A and adjusted for loading control and presented relative to Ang2-alone control. One-way ANOVA; N=3; ^{∗}, ^{∗∗∗} p ≤ 0.05 and 0.001, respectively, relative to Ang2-alone control.

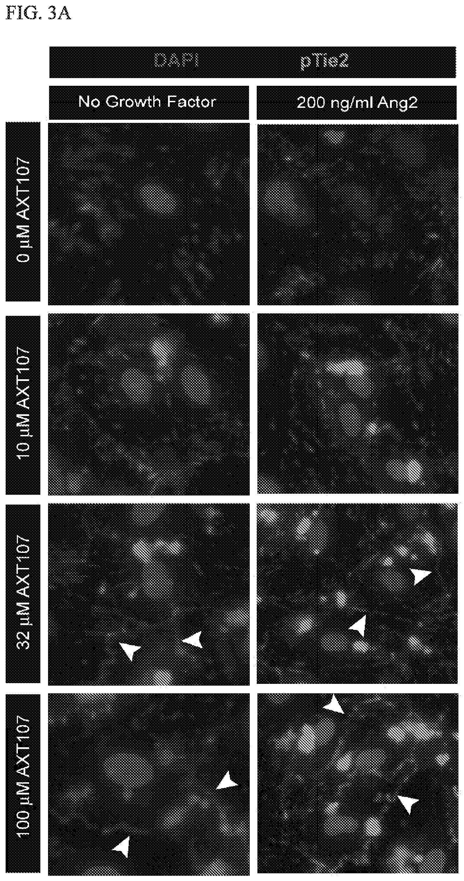

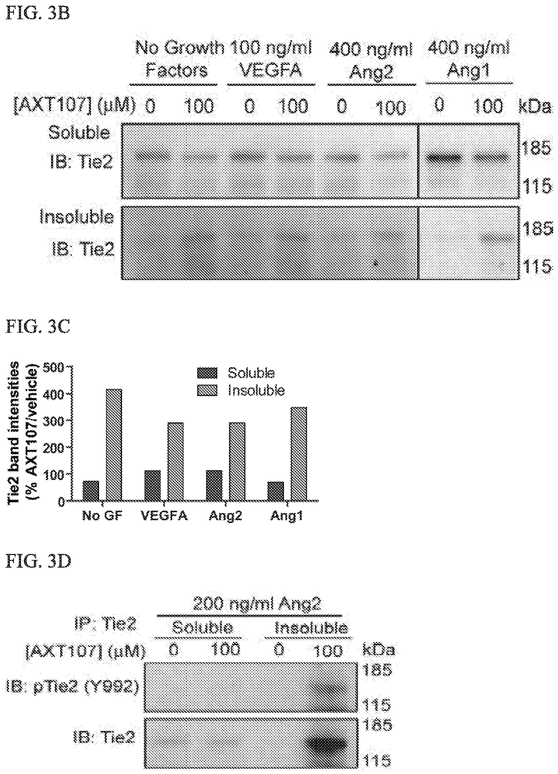

FIG. 3A shows immunofluorescence images of MEC monolayers treated with 0.1% BSA in PBS (left column) or 200 ng/ml Ang2 (right column) for fifteen minutes at varying concentrations of AXT107 and stained for phospho-Tie2 (Y992) (green) and DAPI (blue). White arrows indicate junctional Tie2. FIG. 3B includes western blots of MEC lysates treated with various growth factors and 100 µM AXT107 or DMSO vehicle and fractioned into Triton X-100-soluble and Triton X-100-insoluble pools. FIG. 3C is a graph illustrating densitometric analyses of the western blots disclosed in FIG. 3B; each bar represents the percent change of AXT107-treated samples relative to the corresponding vehicle control of the same growth factor and separated by soluble (left bars) and insoluble (right bars). FIG. 3D is a representative image (n=3) of western blots of Triton X-100-fractionated lysates which were immunoprecipitated for Tie2 and blotted for phospho-Tie2 (top) or for total Tie2 (bottom).

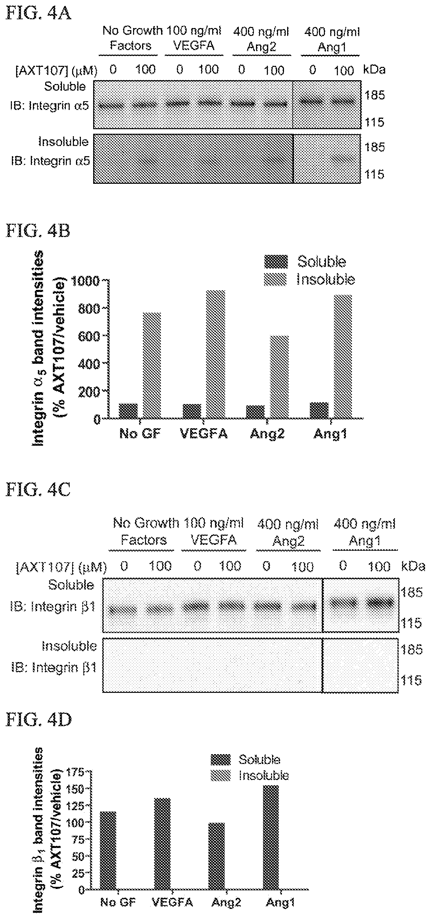

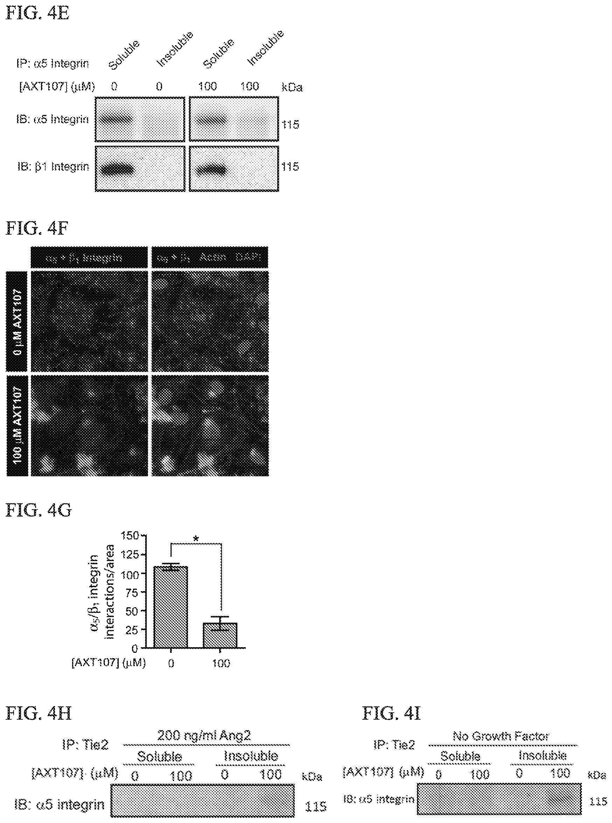

FIGs. 4A and 4C shows western blots of MEC lysates treated with various growth factors and 100 µM AXT107 or DMSO vehicle and fractioned into Triton X-100-soluble and Triton X-100-insoluble pools and immunoblotted for integrin α₅ (A) or immunoblotted for integrin β₁ (C). FIGs. 4B and 4D are graphs illustrating densitometric analyses of bands from, respectively, **FIG. 4A** and **FIG.** 4C; each bar represents the percent change of AXT107-treated samples relative to the corresponding vehicle control of the same growth factor and separated by soluble (left bars) and insoluble (right bars). FIG. 4E shows western blots of Triton X-100-fractionated lysates which were immunoprecipitated for integrin α₅ and blotted for integrin α₅ (top) or for integrin β₁ (bottom). FIG. 4F shows photomicrographs of representative images of a Duolink^{™} assay showing interactions between α₅ and β₁ integrins in MEC monolayers treated with vehicle or with 100 µM AXT107 and FIG. 4G is a graph quantifying the interactions per arbitrary area. **FIGs. 4H** and **4I** show representative images (n=3) of western blots of Triton X-100-fractionated lysates which were immunoprecipitated for Tie2 and blotted for α5 integrin; cells in FIG. 4H were treated with 200 ng/ml of Ang2 and cells in **FIG. 4I** were not treated with a growth factor. N=3.

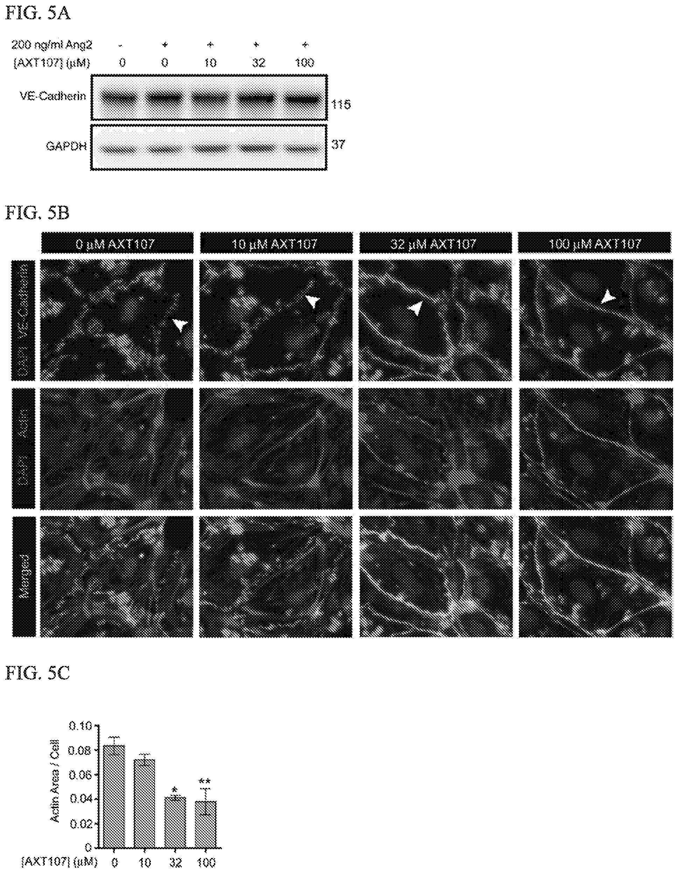

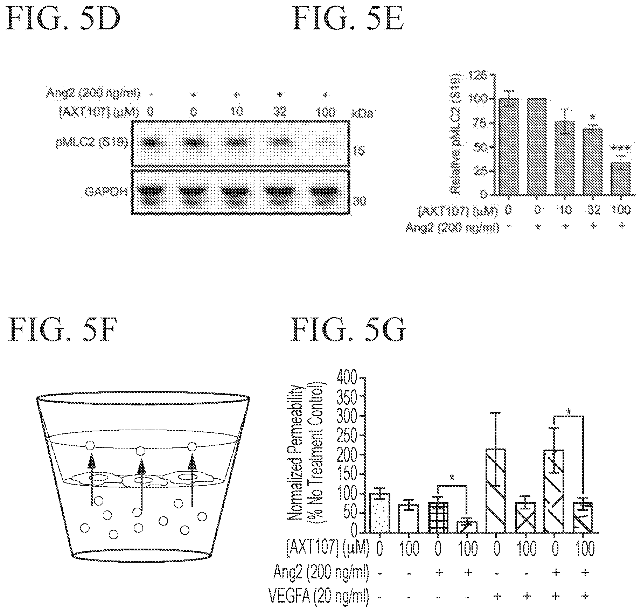

FIG. 5A shows a representative western blot of VE-Cadherin from MEC monolayers treated with 200 ng/ml Ang2 for three hours at various concentrations of AXT107; GAPDH is included as a loading control. FIG. 5B shows photomicrographs of immunofluorescence images of MEC monolayers treated with 200 ng/ml Ang2 and various concentrations of AXT107 that have been stained with antibodies targeting VE-cadherin (green), F-actin (red), and DAPI (blue) and with merged regions shown in yellow; arrows indicate representative regions showing transition of VE-cadherin distribution. FIG. 5C is a graph quantifying the average area of F-actin coverage per cell; one-way ANOVA; N=3; ^{∗}, ^{∗∗} p ≤ 0.05 and 0.01, respectively, relative to Ang2 alone control. FIG. 5D shows representative western blot images of lysates from MECs treated with 200 ng/ml Ang2 and various concentrations of AXT107 blotted against pMLC2 and with GAPDH as a loading control. FIG. 5E is a graph showing a densitometric analysis of the data shown in FIG. 5D; one-way ANOVA; N=3; ^{∗∗∗} p ≤ 0.001 relative to Ang2-alone control. FIG. 5F is a schematic of transendothelial permeability assay described in Example 5. FIG. 5G is a graph showing quantification of FITC-Dextran (40 kDa) migration across MEC monolayers plated on semipermeable substrates following treatment with growth factors and AXT107, where indicated. Student's two-tailed t-test; N≥7; ^{∗} p ≤ 0.05.

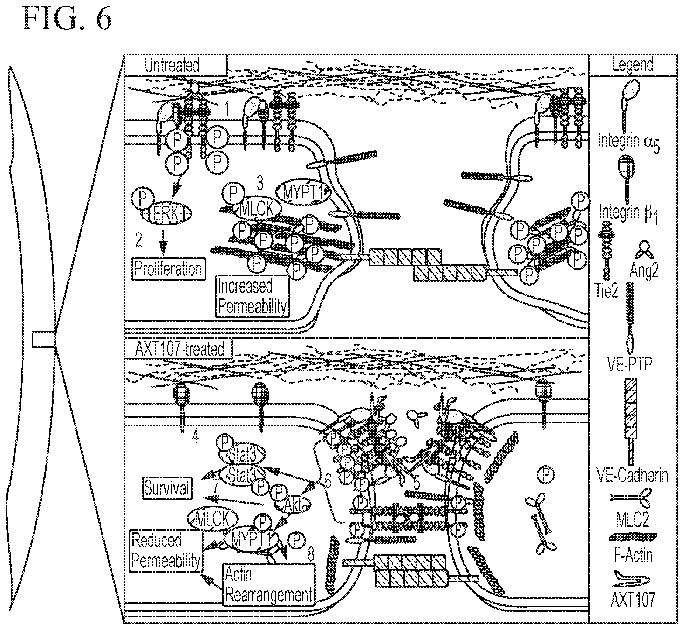

FIG. 6 includes a model for AXT107-mediated activation of Tie2.

### DETAILED DESCRIPTION

The invention is defined by the appended claims. The invention provides formulations for use in treating Tie2-related vascular or lymphatic vessel permeability, by administering one or more collagen IV-derived biomimetic peptide(s). Such peptides can promote the Tie2 agonist activities of Angiopoietin 2 (Ang2), thereby stabilizing vasculature and/or lymphatic vessels.

Collagen IV-derived biomimetic peptides are derived from the α5 fibril of type IV collagen. Disclosed are peptides which comprise, consist of, or consist essentially of the amino acid sequence LRRFSTAPFAFIDINDVINF (SEQ ID NO:1), or derivatives thereof. The peptides may target α5β1 and αVβ3 integrins, and inhibit signaling through multiple receptors, including vascular endothelial growth factor receptor (VEGFR), hepatocyte growth factor receptor (HGFR), insulin-like growth factor receptor (IGFR), and epidermal growth factor receptor (EGFR). As disclosed herein, collagen IV-derived biomimetic peptides further promote Tie2 agonist activities of Angiopoietin 2, thereby stabilizing vasculature and/or lymphatic vessels.

Collagen IV-derived biomimetic peptides include those described in US 9,056,923. Peptides in accordance with the present invention include peptides comprising the amino acid sequence LRRFSTXPXXXXNINNVXNF (SEQ ID NO:2), where X is a standard amino acid or non-genetically encoded amino acid. In some embodiments, X at position 7 is M, A, or G; X at position 9 is F, A, Y, or G; X at position 10 is M, A, G, D-Alanine (dA), or norleucine (Nle); X at position 11 is F, A, Y, G, or 4-chlorophenylalanine (4-ClPhe); X at position 12 and position 18 are independently selected from 2-Aminobutyric acid (Abu), G, S, A, V, T, I, L, or Allylglycine (AllylGly). In various embodiments, the peptide contains about 30 amino acids or less, or about 25 amino acids of less, or about 24 amino acids, or about 23 amino acids, or about 22 amino acids, or about 21 amino acids, or about 20 amino acids. In still other embodiments, one, two, three, four, or five amino acids of SEQ ID NO:2 are deleted.

Disclosed is that the peptide comprises the amino acid sequence LRRFSTAPFAFIDINDVINF (SEQ ID NO:3), or derivative thereof. The peptide of SEQ ID NO:3 is also referred to as AXT107 or as SP2043. Derivatives of the peptide of SEQ ID NO:3 include peptides having from 1 to 5 amino acid substitutions, insertions, or deletions (e.g., 1, 2, 3, 4, or 5 amino acid substitutions, insertions, or deletions collectively) with respect to SEQ ID NO:3, although in some disclosures the Asp at positions 13 and 16 are maintained. It is disclosed that the sequence DINDV is maintained in the derivative. It is disclosed that amino acid substitutions in SEQ ID NO:3 can be at positions occupied by an X at the corresponding position of SEQ ID NO:1. That is, the peptide may have the amino acid sequence of SEQ ID NO:4: LRRFSTXPXXXXDINDVXNF, where X is a standard amino acid or non-genetically encoded amino acid. In some embodiments, X at position 7 is M, A, or G; X at position 9 is F, A, Y, or G; X at position 10 is M, A, G, D-Alanine (dA), or norleucine (Nle); X at position 11 is F, A, Y, G, or 4-chlorophenylalanine (4-ClPhe); X at position 12 and position 18 are independently selected from 2-Aminobutyric acid (Abu), G, S, A, V, T, I, L, or Allylglycine (AllylGly).

Disclosed is that amino acid substitutions are made at any position of a peptide of SEQ ID NO:1, 2, 3, or 4, which can be independently selected from conservative or non-conservative substitutions.It is disclosed that the peptide includes from 1 to 10 amino acids added to one or both termini (collectively). It is disclosed that the N- and/or C-termini may optionally be occupied by another chemical group (other than amine or carboxy, e.g., amide or thiol), and which can be useful for conjugation of other moieties, including PEG or PLGA-PEG co-polymers, as described in further detail herein.

Conservative substitutions may be made, for instance, on the basis of similarity in polarity, charge, size, solubility, hydrophobicity, hydrophilicity, and/or the amphipathic nature of the amino acid residues involved. The 20 genetically encoded amino acids can be grouped into the following six standard amino acid groups:
(1) hydrophobic: Met, Ala, Val, Leu, Ile;
(2) neutral hydrophilic: Cys, Ser, Thr; Asn, Gln;
(3) acidic: Asp, Glu;
(4) basic: His, Lys, Arg;
(5) residues that influence chain orientation: Gly, Pro; and
(6) aromatic: Trp, Tyr, Phe.

As used herein, "conservative substitutions" are defined as exchanges of an amino acid by another amino acid listed within the same group of the six standard amino acid groups shown above. For example, the exchange of Asp by Glu retains one negative charge in the so modified polypeptide. In addition, glycine and proline may be substituted for one another based on their ability to disrupt α-helices. Some preferred conservative substitutions within the above six groups are exchanges within the following sub-groups: (i) Ala, Val, Leu and Ile; (ii) Ser and Thr; (iii) Asn and Gln; (iv) Lys and Arg; and (v) Tyr and Phe.

As used herein, "non-conservative substitutions" are defined as exchanges of an amino acid by another amino acid listed in a different group of the six standard amino acid groups (1) to (6) shown above.

The biomimetic peptide or peptide agent is a peptide of from about 8 to about 30 amino acids, or from about 10 to about 20 amino acids, and has at least 4, at least 5, or at least 6 contiguous amino acids of SEQ ID NO: 1 or 3. In some embodiments, the peptide contains at least one, at least two, or at least three D-amino acids. In some embodiments, the peptide contains from one to about five *(e.g.,* 1, 2, or 3) non-genetically encoded amino acids, which are selected from 2-Aminobutyric acid (Abu), norleucine (Nle), 4-chlorophenylalanine (4-ClPhe), and Allylglycine (AllylGly).

Disclosed are:
LRRFSTMPFMF(Abu)NINNV(Abu)NF (SEQ ID NO:5),
LRRFSTMPAMF(Abu)NINNV(Abu)NF (SEQ ID NO:6),
LRRFSTMPFAF(Abu)NINNV(Abu)NF (SEQ ID NO:7),
LRRFSTMPFMA(Abu)NINNV(Abu)NF (SEQ ID NO:8),
LRRFSTMPF(Nle)F(Abu)NINNV(Abu)NF (SEQ ID NO:9),
LRRFSTMPFM(4-ClPhe)(Abu)NINNV(Abu)NF (SEQ ID NO:10),
LRRFSTMPFMFSNINNVSNF (SEQ ID NO:11),
LRRFSTMPFMFANINNVANF (SEQ ID NO:12),
LRRFSTMPFMFININNVINF (SEQ ID NO:13),
LRRFSTMPFMFTNINNVTNF (SEQ ID NO:14),
LRRFSTMPFMF(AllyGly)NINNV(AllyGly)NF (SEQ ID NO:15),
LRRFSTMPFMFVNINNVVNF (SEQ ID NO:16),
LRRFSTMPFdAFININNVINF (SEQ ID NO:17),
LRRFSTMPFAFININNVINF (SEQ ID NO:18),
LRRFSTAPFAFININNVINF (SEQ ID NO:19),
LRRFSTAPFdAFIDINDVINF (SEQ ID NO:20),
LRRFSTAPFAFIDINDVINW (SEQ ID NO:21),
dLRRdLRRFSTAPFAFIDINDVINF (SEQ ID NO:22),
LRRFSTAPFAFIDINDVINdF (SEQ ID NO:23),
dLRRFSTAPFAFIDINDVINdF (SEQ ID NO:24).
F(Abu)NINNV(Abu)N (SEQ ID NO:25),
FTNINNVTN (SEQ ID NO:26),
FININNVINF (SEQ ID NO:27),
FSNINNVSNF (SEQ ID NO:28),
FANINNVANF (SEQ ID NO:29),
F(AllyGly)NINNV(AllyGly)NF (SEQ ID NO:30),
FVNINNVVNF (SEQ ID NO:31),
FIDINDVINF (SEQ ID NO:32),
FIDINDVINW (SEQ ID NO:33),
FTDINDVTN (SEQ ID NO:34),
A(Abu)NINNV(Abu)NF (SEQ ID NO:35), or
(4-ClPhe)(Abu)NINNV(Abu)NF (SEQ ID NO:36).

It is disclosed that the biomimetic peptides or peptide agents can be chemically synthesized and purified using well-known techniques, such as solid-phase synthesis. See US 9,051,349.

It is disclosed that peptides may be provided in the form of a pharmaceutically acceptable salt in some embodiments, or complexed with other components or encapsulated in particles for targeted or sustained delivery to particular tissues.

The biomimetic peptide or peptide agent of the disclosure is formulated as a pharmaceutically acceptable salt. Pharmaceutically acceptable salts are generally well known to those of ordinary skill in the art, and may include, by way of example, acetate, benzenesulfonate, besylate, benzoate, bicarbonate, bitartrate, bromide, calcium edetate, carnsylate, carbonate, citrate, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, mucate, napsylate, nitrate, pamoate (embonate), pantothenate, phosphate/diphosphate, polygalacturonate, salicylate, stearate, subacetate, succinate, sulfate, tannate, tartrate, or teoclate. Other pharmaceutically acceptable salts may be found in, for example, Remington: The Science and Practice of Pharmacy (20th ed.) Lippincott, Williams & Wilkins (2000). Pharmaceutically acceptable salts include, for example, acetate, benzoate, bromide, carbonate, citrate, gluconate, hydrobromide, hydrochloride, maleate, mesylate, napsylate, pamoate (embonate), phosphate, salicylate, succinate, sulfate, or tartrate.

It is disclosed that the biomimetic peptide or peptide agent may be formulated for local or systemic delivery, for example, using a variety of pharmaceutically acceptable carriers, including, water, saline, dextrose solutions, human serum albumin, liposomes, hydrogels, microparticles and nanoparticles.

In some embodiments, an effective amount of the biomimetic peptide or peptide agent will be within the range of from about 100 to about 1000 µg per dose,. The exact dosage will depend upon, for example, the route of administration, the form in which the compound is administered, and the medical condition and/or patient to be treated. In various embodiments, the peptide is administered no more than once every other month, or no more than once every three months, or no more than once every four months.

The biomimetic peptide or peptide agent is administered by intravitreal injection, for example, for the treatment of diabetic macular edema, retinal vein occlusion, wet age-related macular degeneration (wet AMD), or diabetic retinopathy. A composition comprising the biomimetic peptide or peptide agent may be administered for the treatment of a condition that is refractory or only partially-responsive to VEGF blockade therapy or kinase inhibitor therapy. For example, the biomimetic peptide may be administered after unsuccessful VEGF blockade therapy, and/or may be administered as the primary, first-line therapy (without other agents). In some embodiments, the peptide is administered at a dose of from about 100 µg to about 1000 µg, or in some embodiments, at a dose of from about 200 µg to about 800 µg, or at a dose of from about 400 to about 800 µg. In some embodiments, the dose of the peptide is about 200 µg, about 400 µg, about 500 µg, about 600 µg, about 800 µg, or about 1 mg. The peptide dose is administered once every three months, or once every four months, or once every six months. Because the naked peptide can form a depot upon intravitreal injection, the frequency of dosing can be substantially reduced, with or without formulation into particles. It is disclosed that formulation with microparticles can lead to even less frequent dosing, and in some disclosures the formulation comprises both free and encapsulated protein to provide an initial dose of active agent, with a subsequent, sustained release over several months. Even in the absence of microparticle formulation, intravitreal injection at a frequency of about monthly, or every other month, or once every third month is possible. In some embodiments, the peptide formulation comprises microparticles encapsulating a dose of from 1 mg to about 10 mg of peptide agent, or in some embodiments, a dose of from about 1 mg to 5 mg of peptide, or in some embodiments, a dose of from 1 mg to 3 mg of peptide agent.

The biomimetic peptide is formulated for localized administration. Techniques and formulations generally may be found in Remington: The Science and Practice of Pharmacy (20th ed.) Lippincott, Williams & Wilkins (2000). To aid in bioavailability, it is disclosed that the compositions of the disclosure may be delivered in a nano- or micro-particles, or conjugated to polyethylene glycol or other PK-enhancing conjugates, such as fusion with an antibody Fc domain or albumin amino acid sequence. The agents may be delivered, for example, in a timed- or sustained release form. Techniques for formulation and administration may be found in Remington: The Science and Practice of Pharmacy (20th ed.) Lippincott, Williams & Wilkins (2000). Suitable routes include intraocular (e.g., intravitreal) injections or other modes of delivery.

For injection, the biomimetic peptides or peptide agents may be formulated and diluted in aqueous solutions, such as in physiologically compatible buffers such as Hank's solution, Ringer's solution, or physiological saline buffer.

Disclosed is that the peptide is formulated with a polymeric nanoparticle or microparticle carrier. Disclosed is that the microparticle or nanoparticle comprises a material having one or more degradable linkages, such as an ester linkage, a disulfide linkage, an amide linkage, an anhydride linkage, and a linkage susceptible to enzymatic degradation. Disclosed is that the microparticle or nanoparticle comprises a biodegradable polymer or a blend of polymers selected from the group consisting of poly(lactic-co-glycolic acid) (PLGA), poly(beta-amino ester) (PBAE), polycaprolactone (PCL), polyglycolic acid (PGA), polylactic acid (PLA), poly(acrylic acid) (PAA), poly-3-hydroxybutyrate (P3HB) and poly(hydroxybutyrate-co-hydroxyvalerate). Disclosed is that the particles comprise a blend of PLGA and PBAE. It is disclosed that nondegradable polymers that are used in the art, such as polystyrene, are blended with a degradable polymer or polymers from above to create a copolymer system. Accordingly, disclosed is a nondegradable polymer that is blended with the biodegradable polymer.

Disclosed is a nanoparticle comprising PLGA-PEG copolymers and a conjugated biomimetic peptide. Disclosed is that the conjugated peptide can be a peptide of any one of SEQ ID NOs: 1-36.

Disclosed is that the nanoparticles contain an additional drug or targeting agent conjugated to the surface of the nanoparticle. Disclosed is that the nanoparticles may be made from PLGA-PEG-X and PLGA-PEG-Y polymers, where X is the biomimetic peptide and Y is another drug or targeting agent. Disclosed is that the targeting agent may be a tissue selective targeting agent, or may be selective for a desired cell type, including cancer cells.

Disclosed are other target binding agents that may be used, in addition or alternatively (including alternative integrin-binding moieties), and these include antibodies and antigen-binding portions thereof. The various formats for target binding include a single-domain antibody, a recombinant heavy-chain-only antibody (VHH), a single-chain antibody (scFv), a shark heavy-chain-only antibody (VNAR), a microprotein (cysteine knot protein, knottin), a DARPin, a Tetranectin, an Affibody; a Transbody, an Anticalin, an AdNectin, an Affilin, a Microbody, a peptide aptamer, a phylomer, a stradobody, a maxibody, an evibody, a fynomer, an armadillo repeat protein, a Kunitz domain, an avimer, an atrimer, a probody, an immunobody, a triomab, a troybody, a pepbody, a vaccibody, a UniBody, a DuoBody, a Fv, a Fab, a Fab', a F(ab')2, a peptide mimetic molecule, or a synthetic molecule, or as described in US Patent Nos. or Patent Publication Nos. US 7,417,130, US 2004/132094, US 5,831,012, US 2004/023334, US 7,250,297, US 6,818,418, US 2004/209243, US 7,838,629, US 7,186,524, US 6,004,746, US 5,475,096, US 2004/146938, US 2004/157209, US 6,994,982, US 6,794,144, US 2010/239633, US 7,803,907, US 2010/119446, and/or US 7,166,697. See also, Storz MAbs. 2011 May-Jun; 3(3): 310-317.

Disclosed is that the nanoparticle is synthesized from poly(lactic-co-glycolic acid) polyethylene glycol (PLGA-PEG) block copolymers of tunable size which are covalently linked to the peptide of any one of SEQ ID NOs:1-36, or derivative thereof, or other binding agent as described above. A mix of conjugated and unconjugated polymers in any ratio can be used to create nanoparticles with the desired density of targeting agent on the surface. Disclosed is that the biomimetic peptide comprises the amino acid sequence of SEQ ID NO:3 (referred to as AXT107 or as SP2043).

Disclosed is that the peptide that is conjugated to the particle has the amino acid sequence of SEQ ID NOs:1-36, or derivative thereof. Disclosed is that the nanoparticles are formed from PLGA-PEG-peptide conjugates, or, the peptide is conjugated to preformed particles.

As used herein, the term "nanoparticle," refers to a particle having at least one dimension in the range of about 1 nm to about 1000 nm, including any integer value between 1 nm and 1000 nm (including about 1, 2, 5, 10, 20, 50, 60, 70, 80, 90, 100, 200, 500, and 1000 nm and all integers and fractional integers in between).Disclosed is that the nanoparticle has at least one dimension, e.g., a diameter, of about 50 to about 100 nm. Disclosed is that the nanoparticle has a diameter of about 70 to 100 nm.

Disclosed is that the particle is a microparticle. The term "microparticle" includes particles having at least one dimension in the range of at least about one micrometer (µm). The term "particle" as used herein is meant to include nanoparticles and microparticles.

The particles may be designed to provide desired pharmacodynamic advantages, including circulating properties, biodistribution, and degradation kinetics. Such parameters include size, surface charge, polymer composition, ligand conjugation chemistry, and peptide conjugation density, among others. For example, it is disclosed that the particles have a PLGA polymer core, and a hydrophilic shell formed by the PEG portion of PLGA-PEG co-polymers, wherein a portion of the PLGA-PEG polymers have a terminal attachment of the peptide. The hydrophilic shell may further comprise ester-endcapped PLGA-PEG polymers that are inert with respect to functional groups, such as PLGA-PEG-MeOH polymers. It is disclosed that some or all of the unconjugated polymers have other terminal groups (such as carboxy) to provide fine tuning of the surface properties.

Peptides described herein can be chemically conjugated to the particles using any available process. Functional groups for peptide conjugation include PEG-COOH, PEG-NH2, PEG-SH. See, e.g., Hermanson, BlOCONJUGATE TECHNIQUES, Academic Press, New York, 1996. Activating functional groups include alkyl and acyl halides, amines, sulfhydryls, aldehydes, unsaturated bonds, hydrazides, isocyanates, isothiocyanates, ketones, and other groups known to activate for chemical bonding. Alternatively, peptides can be conjugated through the use of a small molecule-coupling reagent. Examples of coupling reagents include carbodiimides, maleimides, N-hydroxysuccinimide esters, bischloroethylamines, bifunctional aldehydes such as glutaraldehyde, anhydrides and the like.

It is disclosed that the nanoparticles have a core (PLGA) that can be tuned for a specific biodegradation rate *in vivo* (by adjusting the LA:GA ratio and/or molecular weight of the PLGA polymer). It is disclosed that the PLGA is based on a LA:GA ratio of from 20:1 to 1:20, including compositions of L/G of: 5/95, 10/90, 15/85, 20/80, 25/75, 30/70, 35/65, 40/60, 45/55, 50/50, 55/45, 60/40, 65/35, 70/30, 75/25, 80/20, 85/15, 90/10, or 95/5. PLGA degrades by hydrolysis of its ester linkages. The time required for degradation of PLGA is related to the ratio of monomers: the higher the content of glycolide units, the lower the time required for degradation as compared to predominantly lactide units. In addition, polymers that are end-capped with esters (as opposed to the free carboxylic acid) have longer degradation half-lives.

It is disclosed that the PLGA polymers have a molecular weight in the range of about 10K to about 70K, such as about 20K, about 25K, about 30K, about 40K, about 50K, about 60K, or about 70K, to provide tunable particle size. The PEG portion of the polymer is generally in the range of 2K to 5K. It is disclosed that the ratio of PLGA-PEG-peptide and unconjugated PLGA-PEG ranges from about 1:20 to about 20:1, such as from about 1:15 to about 15:1, or about 1:10 to about 10:1, or about 1:5 to about 5:1, or about 1:2 to about 2:1. It is disclosed that the ratio of PLGA-PEG-peptide and unconjugated copolymers is about 1:1. It is disclosed that at least 50% of the polymers have conjugated peptide. Discloses is that the nanoparticle has a size (average diameter) within the range of about 50 to about 200 nm, or within the range of about 50 to about 100 nm. Disclosed is that the nanoparticle has a zeta potential in deionized water within the range of about -5 mV to about -40 mV, and in some embodiments, from about -10 mV to about -30 mV (e.g., about -20, about -25, or about -30 mV).

While the nanoparticle is substantially spherical in somedisclosures, the nanoparticle may optionally be non-spherical.

There are various physical and chemical properties that can affect how a material interacts with a biological system. In the case of microparticle- and nanoparticle-based materials, the choice of material, the size distribution, and the shape distribution of the particles are all critical parameters affecting the particles' activity. It has been previously shown that both the size and shape of a particle can affect the way the particle interacts with various cells of the body. For example, the shape of the particle can affect how well various cell types can uptake the particle, where an ellipsoidal particle is usually more difficult for a cell to uptake than a spherical particle. Stretching the shape of the particles can therefore reduce unwanted uptake of particles, such as by the immune system cells, thereby extending the half-life of the particles in the body. The particle sizes also affect the ability of cells to uptake and interact with the particles. Optimization of the activity of a particle based system can therefore be achieved by tuning the size and shape distribution of the particles.

In some disclosures, the dimensions of the nanoparticle and/or process for stretching the particles as disclosed in WO 2013/086500.

Discloses is that the three-dimensional microparticle or nanoparticle comprises a prolate ellipsoid, wherein the dimension (a) along the x-axis is greater than the dimension (b) along the y-axis, and wherein the dimension (b) along the y-axis is substantially equal to the dimension (c) along the z-axis, such that the prolate ellipsoid can be described by the equation a > b = c. It is disclosed that the ellipsoid is a tri-axial ellipsoid, wherein the dimension (a) along the x-axis is greater than the dimension (b) along the y-axis, and wherein the dimension (b) along the y-axis is greater than the dimension (c) along the z-axis, such that the tri-axial ellipsoid can be described by the equation a > b > c. Disclosed is that the ellipsoid is an oblate ellipsoid, wherein the dimension (a) along the x-axis is equal to the dimension (b) along the y-axis, and wherein the dimension (b) along the y-axis is greater than the dimension (c) along the z-axis, such that the oblate ellipsoid can be described by the equation a = b > c. The presently disclosed asymmetrical particles, however, do not include disclosures in which a = b = c.

Disclosed is that the microparticle or nanoparticle has an aspect ratio ranging from about 1.1 to about 5. It is disclosed the aspect ratio has a range from about 5 to about 10. It is disclosed that the aspect ratio has a range from about 1.5 to about 3.5.

In certain aspects, the invention provides aformulation comprising a peptide for use in preventing or treating a condition involving Tie-2-related vascular permeability or lymphatic vessel integrity in a patient. The formulation comprising a peptide for use comprises administering the collagen IV-derived biomimetic peptide, or nanoparticle formulation thereof, to the patient in an amount effective to reduce Tie-2-dependent vascular or lymphatic permeability. Restoration of Tie2 activation provides therapeutic benefit in conditions associated with edema or vascular permeability, including macular edema, diabetic macular edema (DME), and other conditions, including conditions characterized by acute or chronic inflammation. Tie2-related conditions include diabetic macular edema, retinal vein occlusion, wet AMD, and background diabetic retinopathy,.

In various embodiments, the biomimetic peptide can be delivered for conditions (including macular edema, wet AMD, tumor growth or metastasis) that are refractory or only partially-responsive to vascular endothelial growth factor (VEGF) blockade or inhibitor therapy. Pharmaceutical agents that block VEGF include aflibercept, bevacizumab, ranibizumab, and ramucirumab, and similar agents, which are administered to slow or block angiogenesis. Other agents that target VEGF-mediated biological activity include kinase inhibitors such as pazopanib, sorafenib, sunitinib, axitinib, ponatinib, lenvatinib, vandetanib, regorafenib, and cabozantinib.

Aflibercept is a biopharmaceutical drug for the treatment of wet macular degeneration (EYLEA), and for metastatic colorectal cancer as (ZALTRAP). Aflibercept is an inhibitor of VEGF, and is a recombinant fusion protein consisting of vascular endothelial growth factor (VEGF)-binding portions from the extracellular domains of human VEGF receptors 1 and 2, that are fused to the Fc portion of the human IgG1 immunoglobulin. Aflibercept binds to circulating VEGFs and acts like a "VEGF trap", inhibiting the activity of the vascular endothelial growth factor subtypes VEGF-A and VEGF-B, as well as to placental growth factor (PGF), inhibiting the growth of new blood vessels in the choriocapillaris or the tumor, respectively.

Bevacizumab (AVASTIN) is an angiogenesis inhibitor, a drug that slows the growth of new blood vessels. Bevacizumab is a recombinant humanized monoclonal antibody that blocks angiogenesis by inhibiting VEGF-A. Bevacizumab is administered for treating certain metastatic cancers, including colon cancer, lung cancers (e.g., NSCLC), renal cancers, ovarian cancers, breast cancer, and glioblastoma. Bevacizumab can also be used for treatment of eye diseases, including AMD and diabetic retinopathy.

Ranibizumab (LUCENTIS) is a monoclonal antibody fragment (Fab), and is administered for treatment of wet AMD. The drug is injected intravitreally (into the vitreous humour of the eye) about once a month. Ranibizumab is a monoclonal antibody that inhibits angiogenesis by inhibiting VEGF A, similar to Bevacizumab.

Thus, in some embodiments, the VEGF inhibitor comprises a monoclonal antibody or antigen-binding portion thereof, or comprises extracellular domains of human VEGF receptors 1 and/or 2. For example, the biomimetic peptide may be administered after unsuccessful VEGF blockade therapy, that is, where reductions in angiogenesis, lymphangiogenesis, and/or edema were not observed. In some embodiments, the peptide is administered as an alternative to VEGF blockade therapy. In still further embodiments, the peptide is administered in combination with VEGF blockade therapy, either simultaneously with, before, or after a VEGF blockade regimen. By activating Tie2 signaling, the biomimetic peptides or peptide agents provide therapeutic benefits that may not be observed with VEGF blockage therapy, or VEGF blockade therapy alone.

In some embodiments, the patient has macular edema. Macular edema occurs when fluid and protein deposits collect on or under the macula of the eye (a yellow central area of the retina) and causes it to thicken and swell. The causes of macular edema include chronic or uncontrolled diabetes type 2 *(e.g.,* diabetic retinopathy), in which peripheral blood vessels including those of the retina leak fluid into the retina. Other causes and/or associated disorders include age-related macular degeneration (AMD), chronic uveitis, atherosclerosis, high blood pressure and glaucoma. In some embodiments, the patient has or is at risk of retinal vein occlusion, which can lead to severe damage to the retina and blindness, due to ischemia and edema. The patient receives intra-ocular injection of the peptide formulation, in combination with or as an alternative to VEGF blockade therapy.

The invention provides a peptide composition or formulation. The peptide may have an amino acid sequence of any one of SEQ ID NOs:2-4.The formulation comprises from 100 µg to about 1000 µg of peptide agent per unit dose, and which does not involve encapsulation into particles. Disclosed is that the formulation comprises from about 1 mg to about 10 mg per unit dose (or in some embodiments from 1 to 5 mg or from 1 to 3 mg), and which may comprise particle encapsulation, optionally with free peptide. Also disclosed are formulations providing both encapsulated and free peptide that can provide for an initial dose (e.g., within the range of 100 µg to about 1000 µg), while encapsulated peptide provides a sustained release over several months *(e.g.,* from 3 to 6 months, or more).

As used in this Specification and the appended claims, the singular forms "a," "an" and "the" include plural referents unless the context clearly dictates otherwise.

Unless specifically stated or obvious from context, as used herein, the term "or" is understood to be inclusive and covers both "or" and "and".

Unless specifically stated or obvious from context, as used herein, the term "about" is understood as within a range of normal tolerance in the art, for example, within plus or minus 10%.

The invention will further be described in accordance with the following examples.

### EXAMPLES

Regulatory functions of integrins on Ang-Tie signaling are described in the below examples using an exemplary integrin-binding, biomimetic peptide, AXT107. AXT107 is a twenty-mer peptide, derived from a sequence in type IV collagen. AXT107 binds tightly to integrin α₅β₁ and to integrin αᵥβ₃ and disrupts activities of, at least, the growth factor receptors VEGFR2, cMet, PDGFRβ, and IGF1R (Lee et al., Sci Rep. 2014; 4:7139).

As described herein, AXT107 was found to inhibit vascular leakage by a novel mechanism involving Ang2 and Tie2. AXT107 strongly promotes the agonist activity of Ang2 leading to increased phosphorylation of Tie2, Akt and Stat3 in endothelial cells to strengthen the barrier between endothelial cells in the vasculature. AXT107 disrupts interactions between IGF1R and β1 integrin and enhances VEGFR2 degradation *in vitro* and inhibits the growth and permeability of neovasculature *in vivo.*

The following examples demonstrate that treatment with the exemplary integrin-binding, biomimetic peptide potentiates the normally weak agonistic activity of Ang2 towards Tie2 both *in vitro* and *in vivo* and specifically activates downstream targets associated with EC survival and barrier function. Mechanistically, AXT107 treatment dissociates α₅ integrin and β₁ integrin, resulting in the translocation and activation of Tie2 at EC-EC junctions and decreased monolayer permeability through the reorganization of F-actin and VE-cadherin. Examples that do not fall under the scope of the appended claims are for illustrative purposes only.

### Example 1: AXT107 strongly promotes agonist activity of Ang2

The Tie2 receptor tyrosine kinase signaling pathway and its ligands Angiopoietin1 (Ang1) and Angiopoietin2 (Ang2) regulate vascular permeability, which is compromised in patients with macular edema including patients with retinal vein occlusion (RVO), diabetic macular edema (DME), wet age-related macular degeneration (wet AMD), and background diabetic retinopathy (DR). Ang1 binds Tie2 and stimulates phosphorylation and downstream signaling stabilizing blood vessels (1,2). Ang2 competes with Ang1 for Tie2 binding reducing the phosphorylation of Tie2, and thus it acts as an endogenous Tie2 antagonist (3). Ischemic or hypoxic retina produces high levels of Ang2 (4), and Ang2 levels, like that of VEGF levels, are increased in the eyes of DME patients (5). Ang2 increases the responsiveness of retinal vessels to VEGF and promotes vascular leakage and neovascularization (6-9). These results suggest that restoration of Tie2 activation could provide benefit in conditions associated with edema, including macular edema, DME, and others.

Tie2 may also regulate lymphatic vessel integrity especially during inflammation. Specifically, molecules that enhance phosphorylation of Tie2 could potentially be used to treat lymphatic dysfunction during inflammation (10).

Other data suggest that Ang2 also acts as a weak agonist of Tie2 especially when Ang1 levels are low (11). Exogenous Ang2 activates Tie2 and the promigratory, prosurvival PI3K/Akt pathway in endothelial cells (ECs) but with less potency and lower affinity than exogenous Ang1. ECs produce Ang2 but not Ang1. This endogenous Ang2 maintains Tie2, phosphatidylinositol 3-kinase, and Akt activities, and it promotes EC survival, migration, and tube formation.

AXT107 is an integrin-binding antiangiogenic biomimetic peptide which inhibits signaling from multiple proangiogenic pathways including VEGF, PDGF, HGF, and IGF1; it represents a class of collagen IV-derived biomimetic peptides. Inhibition of these pathways inhibits neovascularization, and inhibition of the VEGF pathway, in particular, inhibits vascular leakage. As described herein, AXT107 was found to inhibit vascular leakage by a novel mechanism involving Ang2 and Tie2. Normally, both VEGF levels and Ang2 levels are increased in patients with DME and they coordinately promote neovascularization and vascular permeability. As shown in FIG. 1, AXT107 (identified in the figure as SP2043) strongly promotes the agonist activity of Ang2 leading to increased phosphorylation of Tie2, Akt and Stat3 in endothelial cells to strengthen the barrier between endothelial cells in the vasculature.

A confluent monolayer of human microvascular endothelial cells (HMEC) was serum starved with EBM-2 overnight. After a 90 min treatment with AXT107 at concentrations of 0, 10, 32, and 100 µM, the cells were treated for 15 min with 1 mM sodium orthovanadate. Next the cells were treated with Angiopoietin-2 (Ang-2) at 200 ng/mL for 15 min. Following cell lysis, the lysates were immunoblotted for pTie2 (Y992), pSTAT3 (Y705), and pAkt (S473).

This result suggests that AXT107 and other peptides from this class could inhibit vascular leakage in patients with DME and other forms of macular edema by simultaneously inhibiting VEGF and other proangiogenic growth factors and by promoting the phosphorylation of Tie2 by increasing the potency of Ang2 as an agonist. This also applies to other diseases where vascular permeability may be important such as cancer, influenza, hemorrhagic fevers, cerebral malaria and others in which edema is a major contributing factor by enhancing the activity of Tie2 signaling.

### Example 2: AXT107 potentiates the activation of Tie2 by Ang2

In this example, a regulatory function of integrins on Ang-Tie signaling was investigated using an exemplary integrin-binding, biomimetic peptide, AXT107.

To investigate the effects of the integrin inhibitor AXT107 on Tie2 signaling, confluent monolayers of microvascular endothelial cells (MECs) on fibronectin-coated dishes were treated with various concentrations of AXT107 followed by exposure to the Tie2 ligands Ang1 or Ang2. Ang1 alone induced phosphorylation of Tie2 (data not shown) whereas Ang2 showed insignificant effects by itself (FIG. 2A and FIG. 2B).

Surprisingly, whereas AXT107 treatment did not significantly influence Ang1 activity (data not shown), a significant, dose-dependent increase in Tie2 phosphorylation was observed in cells treated in combination with AXT107 and Ang2 (FIG. 2A, first row, and FIG. 2B, top left graph). The phosphorylation of the downstream, pro-survival effectors STAT3 and Akt also increased with Ang2 and AXT107 treatment (FIG. 2A, second and third rows, and FIG. 2B, top right and bottom left graphs). However, the phosphorylation of proliferation-associated factor Erk1/2 remained constant in all tested conditions (FIG. 2A, fourth row and FIG. 2B, bottom right graph). For all cases, the total protein levels of Tie2 and the downstream targets remained unchanged (data not shown) and phosphorylation was not induced by peptide alone in absence of either ligand.

Integrin inhibition by AXT107 significantly decreases receptor phosphorylation and downstream signals for many RTKs, e.g., VEGFR2, c-Met, IGF1R, and PDGFRβ, as well as reduced total receptor levels through increased receptor degradation (Lee et al., Sci Rep. 2014; 4:7139). In contrast, AXT107 clearly potentiates the activation of Tie2 by Ang2 both *in vitro* and *in vivo* and does not influence total levels of Tie2, suggesting that increased degradation of the receptor does not occur for Tie2 as it does with other RTKs.

These data demonstrate that treatment with the exemplary integrin-binding, biomimetic peptide potentiates the normally weak agonistic activity of Ang2 towards Tie2, or converts Ang2 from Tie2 antagonist to agonist. Accordingly, AXT107 specifically activates downstream targets associated with endothelial cell (EC) survival and barrier function.

### Example 3: Changes in Tie2 cellular distribution mediated by AXT107 influences receptor activation

The discovery that AXT107 potentiates Ang2-mediated phosphorylation of Akt and STAT3 but does not potentiate ERK1/2 phosphorylation, suggests that AXT107 specifically activates junctional Tie2 rather than Tie2 molecules at the cell-extracellular matrix (ECM) interface. As such, subsequent experiments evaluate the effects of AXT107 at cell-cell junctions in MEC monolayers using immunofluorescence microscopy rather than at the EC-ECM interface.

AXT107 was found to self-assemble into peptide complexes when added to media; this behavior is similar to the depots observed in mouse eyes (data not shown). In samples treated with Ang2 alone (FIG. 3A, top row), phospho-Tie2 was predominantly found in weak, punctate distributions across the cell surface. Samples treated with Ang2 and AXT107 had increased overall fluorescence intensity and redistributed phospho-Tie2 along cell-cell junctions and into large clusters that co-localized with the AXT107 peptide complexes (FIG. 3A, bottom three rows). These large clusters of phospho-Tie2 are unlikely the result of non-specific interactions between the AXT107 peptide complexes and the tested antibodies as no green fluorescence signal was observed for peptide complexes in regions devoid of cells or in wells treated with secondary antibodies alone (data not shown). Previous reports have emphasized the importance of clustering in Ang2's activation of Tie2 and may explain the potentiation of Tie2 phosphorylation by Ang2 and the activation of downstream effectors of vessel stability and quiescence.

Tie2 at EC-EC junctions form actin-rich complexes that are insoluble in Triton X-100-based lysis buffers but are soluble when distributed over the surface of the cell. Therefore, MEC monolayers were treated with various combinations of AXT107, Ang1, and Ang2 and cell lysates were fractionated by their solubility in Triton X-100-based lysis buffers. Experiments including VEGF₁₆₅ were also performed since VEGFR2 signaling often opposes the activities of Tie2. In each experiment, 100 µM AXT107 was used since it provided clear results relative to lower concentrations of AXT107.

Consistent with the observations from the immunofluorescence assays (as shown in FIG. 3A), increased amounts of Tie2 were found in the insoluble fraction of lysates treated with AXT107; the increased amounts were independent of the specific growth factor treatment (FIG. 3B and 3C). Similar results were also obtained for Tiel (data not shown), a co-receptor recently shown to be essential for the activation of junctional Tie2 (Korhonen et al., J Clin Invest. 2016; 126(9):3495-3510).

Next, experiments were performed to determine whether or not relocation of Tie2 to the insoluble fraction was important for Tie2's activation by Ang2. Tie2 was immunoprecipitated from fractionated MEC lysates exposed to Ang2 with or without AXT107 and then immunoblotted for phospho-Tie2. Interestingly, phosphorylation was observed only in the insoluble fractions of peptide-treated samples (FIG. 3D).

These data demonstrate that treatment with AXT107 peptide results in the translocation of Tie2 to EC-EC junctions and activation of Tie2.

### Example 4: AXT107 disrupts interactions between α5 and β1 integrin subunits

α₅β₁-integrin heterodimer and αᵥβ₃-integrin heterodimer are primary targets of AXT107. To investigate the possibility of an integrin-mediated mechanism for regulating Tie2, fractionated MEC lysates immunoblotted for the α₅ integrin subunit revealed that a portion of the α₅ integrin subunit relocated to the insoluble fraction in samples treated with AXT107 (FIG. 4A and 4B); this result is similar to what was observed for both Tie2 (FIG. 3D) and Tiel (data not shown).

Surprisingly, the β₁ integrin subunit was never observed in the insoluble fraction despite the use of long exposure times and high antibody concentrations (FIG. 4C to 4E). This suggests treatment with AXT107 disrupts the interaction between the α₅ integrin subunit and β₁ integrin subunit in an integrin heterodimer. Since β₁ integrin is the only known β subunit to heterodimerize with the α₅ integrin subunit, it unlikely that the α₅ subunit, that was observed here in the insoluble fractions, originated from a heterodimeric integrin pair other than α₅β₁. Unfortunately, high background impaired the visualization of α₅ integrin alone by immunofluorescence. Given the unexpected discovery that AXT107 dissociates the integrin heterodimer, this discovery was confirmed in several independent assays. Immunoprecipitation of α₅ integrin in Triton X-100 fractionated lysates revealed that while α₅ integrin could be observed in the insoluble fraction after AXT107 treatment, interactions with β₁ were only found in the soluble fractions (data not shown). Thus, interactions between Tie2 and heterodimerized α₅β₁ integrin appear to retain Tie2 at the EC-ECM interface. This is consistent with reports that Tie2 does not interact with β1 integrin in absence of the α5 subunit (Cascone et al., J Cell Biol. 2005; 170(6):993-1004); disruption of these integrin heterodimers allow for the formation of Tie2-containing complexes at EC-EC junctions and in large clusters.

Additionally, changes in the interaction between the α₅ and β₁ subunits were further investigated using Duolink^{™} technology which can visualize individual interactions between α₅ and β₁ integrin subunits as distinct spots by fluorescence microscopy. Consistent with the results from the Triton X-100 fractionation studies (FIGs. 4A to 4E), interactions between α₅ and β₁ integrin subunits were significantly reduced in monolayers treated with AXT107 compared to vehicle alone **(****FIG. 4F** and **FIG.** 4G).

Finally, it was determined whether or not the α₅ integrin subunit remains complexed with Tie2 following its disassociation with β₁ integrin. As shown in FIG. 4H and FIG. 4I, α₅ integrin was observed in the peptide-treated, insoluble fraction following immunoprecipitation of Tie2.

These data demonstrate that treatment with the AXT107 peptide dissociate α5 integrin and β1 integrin subunits from a heterodimer.

Interestingly, the knockdown of β1 integrin has been shown to decrease Akt phosphorylation; this contrasts the results from α5 integrin knockdown or peptide treatment. As a possible explanation, β1 is the most promiscuous of the integrins and decreases in its protein levels may impact more cellular activities, both Tie2-dependent and Tie2-independent, in comparison to knockdowns of the relatively-specific α5 integrin or conditions in which integrin levels remain constant but are inhibited. Taken together, these findings emphasize the importance of integrins in the preferential activation of signaling pathways downstream of Tie2.

### Example 5: Treatment with AXT107 strengthens and narrows endothelial cell junctions

Having demonstrated that AXT107 potentiates the activation of Tie2 through the disruption of interactions between subunits in α₅β₁ integrin, the following experiments were performed to determine the functional consequence of this activity.

Tie2 signaling is a major regulator of vascular permeability and dysfunction in this activity is known to contribute to increased macular edema and disease progression. Specifically, Tie2 strengthens cell-cell junctions through the formation of *trans* interactions with Tie2 receptors on adjacent cells and the reorganization of VE-Cadherin complexes continuously along cell-cell junctions.

Consistent with previous reports of Tie2 activation by Ang1, the total level of VE-cadherin remained unchanged after three hours of AXT107 and Ang2 treatment (FIG. 5A). However, immunofluorescence imaging revealed clear changes in the structure of VE-cadherin junctions. As shown in FIG. 5B, at lower concentrations, the distribution of VE-cadherin was discontinuous and jagged in appearance but became progressively smoother with increasing concentrations of AXT107. The jaggedness of these junctions is related to the structure of actin within the cell. Absent AXT107 treatment (FIG. 5B, left column), radial actin fibers were arranged across the cells but became more cortical with increasing concentrations of AXT107 (compare with remaining columns in FIG. 5B; *see,* also, FIG. 5C). Radial actin functions to pull cells apart to increase permeability whereas junctional actin does not exert the same pull and thus results in decreased vascular permeability.

Phosphorylation of Tie2 is known to stimulate the Rap1-GTPase pathway, leading to a reduction in the phosphorylation of the downstream motor protein myosin light chain 2 (MLC2) associated with actin rearrangement. As shown in FIG. 5D, phosphorylation of MLC2 is reduced in a dose-dependent manner following treatment with AXT107 and Ang2.

The reorganization of VE-cadherin, actin, and Tie2 at endothelial cell junctions by AXT107 suggests that treatment with the peptide stabilizes cell-cell interactions. The integrity of these junctions is also important for the regulation of monolayer permeability by controlling the size of intercellular openings. The effect of the AXT107 on EC permeability was further investigated by the transendothelial diffusion of FITC-labeled dextran across MEC monolayers seeded onto permeable Transwell^{®} substrates. A schematic of the assay is shown in FIG. 5F.

As shown in FIG. 5G, treatment with Ang2 or AXT107 alone influenced FITC-dextran diffusion across the monolayer whereas VEGF treatment appeared to increase permeability (although non-significantly) alone or in combination with Ang2. Interestingly, the addition of Ang2 alone or in combination with VEGF to monolayers pre-incubated with 100 µM AXT107 showed a significant decrease in FITC-dextran diffusion into the top chamber when compared to cells treated with the growth factors alone. A similar, but non-significant trend was also observed between monolayers treated in VEGF in the presence and absence of peptide.

These data demonstrate that treatment with the AXT107 peptide results in decreased monolayer permeability through the reorganization of F-actin and VE-cadherin.

The importance of integrin interactions in the regulation of Tie2 signaling suggests that natural mechanisms may exist for this to occur within organisms. The treatment of ECs with cartilage oligomeric matrix protein (COMP)-Angl has been shown to induce junctional relocation of Tie2, suggesting that it may stimulate Tie2's dissociation from β1 integrin through a yet-unknown mechanism. Interestingly, differences in the C-termini of Ang1 and Ang2 were found to alter their interactions with β1 integrin which, consequently, could only be activated by Ang2.

Without wishing to be bound by theory, the data disclosed herein provide a model for AXT107-mediated activation of Tie2. As illustrated in FIG. 6, top, in the absence of AXT107 (1) Ang2 weakly activates Tie2 in complex with integrin α₅β₁ heterodimers at the EC-ECM interface, which (2) preferentially activates proliferative signals (e.g., ERK1/2). (3) Active MLC kinase (MLCK) activates MLC3 and leads to formation of radial actin stress fibers within the cell and tension at EC-EC cell junctions. However, in the presence of AXT107, in FIG. 6, bottom, (4) α₅ integrin separates from β₁ integrin and (5) migrates to EC-EC junctions along with Tie2 to form large complexes and/or trans-interactions across junctions. (6) These complexes potentiate the phosphorylation of Tie2 and activate (7) Akt- and STAT3-mediated survival pathways. Additionally, (8) MLC phosphatase is activated *via* a RAP1 or RAC1 pathway, which leads to reduced MLC2 activity, increased cortical actin, and stabilized junctions.

### Example 6: In vivo, the exemplary integrin-binding, biomimetic potentiates Tie2 phosphorylation

To investigate the effects of AXT107 on Tie2 activation *in vivo,* retinopathy of prematurity (ROP) mouse model was used. In this system, P7 pups are placed in 75% O₂ for five days, resulting in a loss of retinal capillary density from hyperoxia and a rapid induction of neovascularization upon the pup's return to normoxic conditions. Here, the *in vivo* potential of the peptide was demonstrated using an eye vasculature model. Increased levels of Ang2 in the vitreous contribute to vessel leakage and macular edema in various retinopathies.

### The following exemplary methods and materials were used in the above examples:

### Cell culture and Reagents

Human dermal microvascular endothelial cells (Lonza) were maintained at 37 °C and 5% CO₂ in EBM-2MV medium (Lonza) and used between passages two through seven. Where applicable, cells were serum starved in EBM-2 medium (Lonza) with no supplements. For FITC-Dextran permeability assays phenol red-free media were used to avoid auto-fluorescence. AXT107 were manufactured at New England Peptide by solid state synthesis, lyophilized, and dissolved in 100% DMSO. After dilution, preferably, DMSO concentrations did not exceed 0.25%.

### Western Blotting

For Ang1/2 signaling investigations, cell culture dishes (10 cm diameter) were coated with 5 µg/ml fibronectin (FN1; Sigma-Aldrich, St. Louis, MO) for two hours at 37 °C. The FN1 solution was then removed by aspiration and 5×10⁶ microvascular endothelial cells (MECs; Lonza, Walkersville, MD) were plated in EGM-2MV media (Lonza) and cultured for forty-eight hours at 37 °C. The cells were then serum starved for sixteen hours in serum-free EGM-2 base media (Lonza). AXT107 (0-100 µM, as indicated) was subsequently added to each culture and incubated for seventy-five minutes at 37 °C. The cultures were then treated with 1 mM sodium vanadate (New England Biolabs, Ipswich, MA) for fifteen minutes to enhance the phospho-Tie2 signal followed by stimulation with 200 ng/ml angiopoietin (R&D Systems, Minneapolis, MN) for an additional fifteen minutes. The cells were then transferred to ice, washed twice with ice cold Dulbecco's phosphate-buffered saline (dPBS) containing Ca²⁺ and Mg²⁺, and collected by scraping in 500 µl of 1x Blue Loading Buffer (Cell Signaling, Danvers, MA). Lysate samples were then sonicated, boiled, and resolved by SDS-PAGE. Specific proteins were identified by Western blot, using the following primary antibodies: Cell Signaling - phospho-Tie2 (Y992) (Cat#: 4221), Tie2 (Cat#: 7403), phospho-Stat3 (Y705) (Cat#: 4113), Stat3 (Cat#: 4904), phospho-Akt (S473) (Cat#: 4058), Akt (Cat#: 9272), phospho-p44/42 MAPK (T202/Y204) (Cat#: 4370), p44/42 MAPK (Cat#: 4695); BD Transduction Laboratories - β₁ integrin (Cat#: 610467); Millipore - α₅ integrin (Cat#: AB1928) and detected with HRP-conjugated goat anti-rabbit and sheep anti-mouse secondary antibodies (GE healthcare).

### Triton X-100 Fractionation

The isolation of Triton X-100 soluble and insoluble fractions was performed using modifications to previously-described procedures *(see, e.g.,* Lampugnani et al., J Cell Biol. 1995; 129(1):203-217). FN1-coated six-well plates were seeded with 2.5×10⁶ cells and cultured for forty-eight hours, as described above. The cultures were then serum starved for ninety minutes in EBM-2 media, treated with 100 µM AXT107 or DMSO vehicle, and fifteen minutes with 1 mM sodium vanadate. The cells were then stimulated with either 100 ng/ml VEGFA, 400 ng/ml Ang2, or PBS for fifteen minutes. The plates were then transferred to ice and washed twice with cold dPBS containing Ca²⁺ and Mg²⁺ and twice with EBM-2 media. The media was then removed and the cells incubated for thirty minutes on ice, at 4 °C in 200 µl of Triton X-100 extraction buffer (10 mM Tris-HCl, pH 7.5; 150 mM NaCl; 2 mM CaCl₂; 1% NP-40; 1% Triton C-100; and a protease inhibitor cocktail (Cell Signaling, Cat#: 5871)) with occasional agitation. The extraction buffer was gently collected and centrifuged at 12,000 x g for five minutes. The supernatant was then mixed with 125 µl of 3x Blue Loading Dye, boiled, and saved as the Triton X-100 soluble fraction at -20 °C. The remaining insoluble fraction was washed twice with wash buffer (10 mM Tris-HCl, pH 7.5; 150 mM NaCl; cOmplete^{™} Mini protease inhibitor tablets (Roche)) and collected in 375 µl of 1x Blue Loading Dye with scraping followed by centrifugation and boiling, as described above. This lysate was saved at -20 °C as the Triton X-100 insoluble fraction. Samples were analyzed by western blot as described above.

For pull-down variations, insoluble fractions were instead collected in RIPA buffer (Sigma) treated with a protease and phosphatase inhibitor cocktail (Cell Signaling) and 5 mM EDTA. Lysates were sonicated briefly and incubated for one hour with anti-Tie2 (Cell Signaling, Cat#: 4224) or anti-α₅ integrin (Millipore; Cat#: AB1928) with end-over-end mixing. Subsequently, 20µl of Protein Agarose A/G beads (Santa Cruz) were added and the samples incubated for another hour. Beads were collected by centrifugation at 1,500 x g and 4 °C, washed four times with PBS, resuspended in SDS-based Blue Loading Dye (Cell Signaling), boiled and resolved by SDS-PAGE.

### Immunofluorescence

Glass-bottomed, 96-well plates with half well size were coated with 10 µg/ml FN1 for two hours at 37 °C. The FN1 solution was then removed by aspiration and the plate seeded with 4×10³ MECs in EGM-2MV media. After twenty-four hours, the cells were washed once with dPBS containing Ca²⁺ and Mg²⁺ to remove dead cells and the cells were allowed to grow for an additional twenty-four hours. For three-hour duration treatments (*i.e.,* VE-cadherin), cells were washed twice with dPBS containing Ca²⁺ and Mg²⁺ and serum starved in EBM-2 for ninety minutes. The media was then removed and the cells were treated for three hours with 100 µl of EBM-2 media containing 200 ng/ml Ang2 or PBS and varying concentrations of AXT107 or DMSO. For fifteen minute-treated samples *(i.e.,* phospho-Tie2), the cells were serum starved in EBM-2 media for 165 minutes, incubated for ninety minutes with varying concentrations of AXT107 or DMSO in EBM-2, and finally stimulated for fifteen minutes with 200 ng/ml Ang2 or PBS supplemented with peptide to retain the same concentrations. These times were chosen so that both treatment procedures would be completed at the same time. The cells were then washed twice with cold dPBS containing Ca²⁺ and Mg²⁺ and fixed in 10% neutral buffered formalin for fifteen minutes. The formalin solution was then removed, the wells washed three times in dPBS containing Ca²⁺ and Mg²⁺. The cells were then blocked in blocking buffer (5% normal goat serum; 0.3% Triton X-100 in dPBS containing Ca²⁺ and Mg²⁺) and stained for sixteen hours with primary antibodies for phospho-Tie2 (Y992) (R&D Systems; Cat#: AF2720) or VE-cadherin (Cell Signaling; Cat#: 2500) diluted 1:150 in antibody dilution buffer (1% BSA; 0.3% Triton X-100 in dPBS containing Ca²⁺ and Mg²⁺). The wells were then washed three times with dPBS and incubated for one hour with Alexafluor 488-conjugated goat anti-rabbit secondary antibodies (Cell Signaling; Cat#: 4412) diluted 1:300 in antibody dilution buffer. The wells were then washed twice and stained for twenty minutes with Alexafluor 555-conjugated phalloidin (Cell Signaling; Cat#: 8953) diluted 1:20 in PBS. The cells were then washed twice again in dPBS, stained with DAPI for twenty minutes, and solution exchanged with dPBS for imaging. Cells were imaged using the BD Pathway 855 system and Attovision software (BD Biosciences).

### Duolink Protein Interaction Analysis

Glass bottom, 96-well plates were coated with FN1, seeded with MECs, as described above for the immunofluorescence experiments. After growing for forty-eight hours, cells were serum starved for three hours, treated with 100 µM AXT107 or DMSO vehicle for ninety minutes, washed twice with dPBS containing Ca²⁺ and Mg²⁺, and fixed in 10% neutral buffered formalin. Cells were blocked in 5% normal goat serum; 0.3% Triton X-100 in dPBS containing Ca²⁺ and Mg²⁺ for one hour and incubated overnight at 4 °C with rabbit anti-α5 integrin and mouse anti-β1 integrin antibodies in PBS containing Ca²⁺ and Mg²⁺ with 1% BSA and 0.1% Triton X-100. Interaction spots were developed using DUOLINK green detection reagent according to the manufacturer's instructions and detected using the BD pathway 855 system.

### FITC transwell permeability assay

Transwell, twenty-four-well inserts (Corning) were coated with 7.5 µg/cm² FN1 for two hours at 37 °C, aspirated, and then dried for thirty minutes at room temperature. Wells were then seeded with 7.5×10⁴ MECs in 100 µl of EBM-2 media (without phenol red) and allowed to settle for thirty minutes at room temperature. 1 ml of EGM-2 media was then added to the bottom chamber and an additional 200 µl to the top chamber. The plate was incubated for twenty-four hours at 37 °C after which the media was aspirated and an additional 7.5×10⁴ MECs were plated in each well as described above. After forty-eight hours at 37 °C, the media was aspirated from both chambers and the cells were washed twice in dPBS containing Ca²⁺ and Mg²⁺, once with EBM-2 media (without phenol red) and serum starved in EBM-2 media applied to both chambers for two hours at 37 °C. After this incubation, 100 µM AXT107 or an equivalent amount of DMSO vehicle was added and incubated for an additional ninety minutes. In the top chamber, the cells were then treated with 200 ng/ml Ang2, 100 ng/ml VEGFA, both, or PBS control and in the bottom chamber, the cells were then treated with 25 µg/ml FITC-Dextran (40 kDa MW). AXT107 was also added in both chambers to maintain a concentration of 100 µM. After three hours, 10 µl was removed from the top chamber of each well and mixed with 90 µl of water in a clear bottom, 96-well plate. Fluorescence values for each sample were calculated using a Perkin Elmer plate reader.

### REFERENCES

Eklund L, Kangas J and Saharinen P. Angiopoietin-Tie signalling in the cardiovascular and lymphatic systems. Clin Sci (Lond). 2017; 131(1):87-103.

Saharinen P, Eklund L and Alitalo K. Therapeutic targeting of the angiopoietin-TIE pathway. Nat Rev Drug Discov. 2017.

Davis S, Aldrich TH, Jones PF, Acheson A, Compton DL, Jain V, Ryan TE, Bruno J, Radziejewski C, Maisonpierre PC and Yancopoulos GD. Isolation of angiopoietin-1, a ligand for the TIE2 receptor, by secretion-trap expression cloning. Cell. 1996; 87(7):1161-1169.

Saharinen P, Eklund L, Miettinen J, Wirkkala R, Anisimov A, Winderlich M, Nottebaum A, Vestweber D, Deutsch U, Koh GY, Olsen BR and Alitalo K. Angiopoietins assemble distinct Tie2 signalling complexes in endothelial cell-cell and cell-matrix contacts. Nat Cell Biol. 2008; 10(5):527-537.

Frye M, Dierkes M, Kuppers V, Vockel M, Tomm J, Zeuschner D, Rossaint J, Zarbock A, Koh GY, Peters K, Nottebaum AF and Vestweber D. Interfering with VE-PTP stabilizes endothelial junctions in vivo via Tie-2 in the absence of VE-cadherin. J Exp Med. 2015; 212(13):2267-2287.

Dalton AC, Shlamkovitch T, Papo N and Barton WA. Constitutive Association of Tiel and Tie2 with Endothelial Integrins is Functionally Modulated by Angiopoietin-1 and Fibronectin. PLoS One. 2016; 11(10):e0163732.

Fiedler U, Scharpfenecker M, Koidl S, Hegen A, Grunow V, Schmidt JM, Kriz W, Thurston G and Augustin HG. The Tie-2 ligand angiopoietin-2 is stored in and rapidly released upon stimulation from endothelial cell Weibel-Palade bodies. Blood. 2004; 103(11):4150-4156.

Maisonpierre PC, Suri C, Jones PF, Bartunkova S, Wiegand SJ, Radziejewski C, Compton D, McClain J, Aldrich TH, Papadopoulos N, Daly TJ, Davis S, Sato TN and Yancopoulos GD. Angiopoietin-2, a natural antagonist for Tie2 that disrupts in vivo angiogenesis. Science. 1997; 277(5322):55-60.

Benest AV, Kruse K, Savant S, Thomas M, Laib AM, Loos EK, Fiedler U and Augustin HG. Angiopoietin-2 is critical for cytokine-induced vascular leakage. PLoS One. 2013; 8(8):e70459.

Tabruyn SP, Colton K, Morisada T, Fuxe J, Wiegand SJ, Thurston G, Coyle AJ, Connor J and McDonald DM. Angiopoietin-2-driven vascular remodeling in airway inflammation. Am J Pathol. 2010; 177(6):3233-3243.

Daly C, Pasnikowski E, Burova E, Wong V, Aldrich TH, Griffiths J, Ioffe E, Daly TJ, Fandl JP, Papadopoulos N, McDonald DM, Thurston G, Yancopoulos GD and Rudge JS. Angiopoietin-2 functions as an autocrine protective factor in stressed endothelial cells. Proc Natl Acad Sci U S A. 2006; 103(42):15491-15496.

Yuan HT, Khankin EV, Karumanchi SA and Parikh SM. Angiopoietin 2 is a partial agonist/antagonist of Tie2 signaling in the endothelium. Mol Cell Biol. 2009; 29(8):2011-2022.

Korhonen EA, Lampinen A, Giri H, Anisimov A, Kim M, Allen B, Fang S, D'Amico G, Sipila TJ, Lohela M, Strandin T, Vaheri A, Yla-Herttuala S, Koh GY, McDonald DM, Alitalo K, et al., Tiel controls angiopoietin function in vascular remodeling and inflammation. J Clin Invest. 2016; 126(9):3495-3510.

Shen J, Frye M, Lee BL, Reinardy JL, McClung JM, Ding K, Kojima M, Xia H, Seidel C, Lima e Silva R, Dong A, Hackett SF, Wang J, Howard BW, Vestweber D, Kontos CD, et al., Targeting VE-PTP activates TIE2 and stabilizes the ocular vasculature. J Clin Invest. 2014; 124(10):4564-4576.

Singh H, Milner CS, Aguilar Hernandez MM, Patel N and Brindle NP. Vascular endothelial growth factor activates the Tie family of receptor tyrosine kinases. Cell Signal. 2009; 21(8):1346-1350.

Cascone I, Napione L, Maniero F, Serini G and Bussolino F. Stable interaction between alpha5beta1 integrin and Tie2 tyrosine kinase receptor regulates endothelial cell response to Ang-1. J Cell Biol. 2005; 170(6):993-1004.

Lee E, Lee SJ, Koskimaki JE, Han Z, Pandey NB and Popel AS. Inhibition of breast cancer growth and metastasis by a biomimetic peptide. Sci Rep. 2014; 4:7139.

Karagiannis ED and Popel AS. A systematic methodology for proteome-wide identification of peptides inhibiting the proliferation and migration of endothelial cells. Proc Natl Acad Sci U S A. 2008; 105(37):13775-13780.

Chen TT, Luque A, Lee S, Anderson SM, Segura T and Iruela-Arispe ML. Anchorage of VEGF to the extracellular matrix conveys differential signaling responses to endothelial cells. J Cell Biol. 2010; 188(4):595-609.

Soldi R, Mitola S, Strasly M, Defilippi P, Tarone G and Bussolino F. Role of alphavbeta3 integrin in the activation of vascular endothelial growth factor receptor-2. EMBO J. 1999; 18(4):882-892.

Veevers-Lowe J, Ball SG, Shuttleworth A and Kielty CM. Mesenchymal stem cell migration is regulated by fibronectin through alpha5betal-integrin-mediated activation of PDGFR-beta and potentiation of growth factor signals. J Cell Sci. 2011; 124(Pt 8):1288-1300.

Rahman S, Patel Y, Murray J, Patel KV, Sumathipala R, Sobel M and Wijelath ES. Novel hepatocyte growth factor (HGF) binding domains on fibronectin and vitronectin coordinate a distinct and amplified Met-integrin induced signalling pathway in endothelial cells. BMC Cell Biol. 2005; 6(1):8.

Baron V and Schwartz M. Cell adhesion regulates ubiquitin-mediated degradation of the platelet-derived growth factor receptor beta. J Biol Chem. 2000; 275(50):39318-39323.

Campochiaro PA, Khanani A, Singer M, Patel S, Boyer D, Dugel P, Kherani S, Withers B, Gambino L, Peters K, Brigell M and Group T-S. Enhanced Benefit in Diabetic Macular Edema from AKB-9778 Tie2 Activation Combined with Vascular Endothelial Growth Factor Suppression. Ophthalmology. 2016; 123(8):1722-1730.

Orfanos SE, Kotanidou A, Glynos C, Athanasiou C, Tsigkos S, Dimopoulou I, Sotiropoulou C, Zakynthinos S, Armaganidis A, Papapetropoulos A and Roussos C. Angiopoietin-2 is increased in severe sepsis: correlation with inflammatory mediators. Crit Care Med. 2007; 35(1):199-206.

Ziegler T, Horstkotte J, Schwab C, Pfetsch V, Weinmann K, Dietzel S, Rohwedder I, Hinkel R, Gross L, Lee S, Hu J, Soehnlein O, Franz WM, Sperandio M, Pohl U, Thomas M, et al., Angiopoietin 2 mediates microvascular and hemodynamic alterations in sepsis. J Clin Invest. 2013.

Han S, Lee SJ, Kim KE, Lee HS, Oh N, Park I, Ko E, Oh SJ, Lee YS, Kim D, Lee S, Lee DH, Lee KH, Chae SY, Lee JH, Kim SJ, et al., Amelioration of sepsis by TIE2 activation-induced vascular protection. Sci Transl Med. 2016; 8(335):335ra355.

Lampugnani MG, Corada M, Caveda L, Breviario F, Ayalon O, Geiger B and Dejana E. The molecular organization of endothelial cell to cell junctions: differential association of plakoglobin, beta-catenin, and alpha-catenin with vascular endothelial cadherin (VE-cadherin). J Cell Biol. 1995; 129(1):203-217.

## Claims

1. A formulation comprising a peptide, wherein the peptide comprises an amino acid sequence with the formula LRRFSTXPXXXXDINDVXNF (SEQ ID NO: 4) or LRRFSTXPXXXXNINNVXNF (SEQ ID NO: 2), where X is a standard amino acid or non-genetically encoded amino acid,
wherein the formulation comprises from 100 µg to about 1000 µg of the peptide agent, wherein the formulation does not involve encapsulation into particles,
for use in the treatment of diabetic macular edema, retinal vein occlusion, wet age-related macular degeneration (wet AMD), or diabetic retinopathy, by intravitreal injection wherein the frequency of administration is no more frequent than about once every three months.

2. The formulation for the use of claim 1, wherein the frequency of administration is once every four months.

3. The formulation for the use of claim 1, wherein the frequency of administration is once every six months.

4. The formulation for the use of claim 1, wherein the dose of the peptide is from about 200 µg to about 800 µg.

5. The formulation for the use of claim 1, wherein the dose of the peptide is from about 400 µg to about 800 µg.

6. The formulation for the use of any one of claims 1 to 3, wherein the X at position 7 of SEQ ID NO: 4 or SEQ ID NO: 2 is M, A, or G; X at position 9 of SEQ ID NO: 4 or SEQ ID NO: 2 is F, A, Y, or G; X at position 10 of SEQ ID NO: 4 or SEQ ID NO: 2 is M, A, G, D-Alanine (dA), or norleucine (Nle); X at position 11 of SEQ ID NO: 4 or SEQ ID NO: 2 is F, A, Y, G, or 4-chlorophenylalanine (4-ClPhe); X at position 12 and position 18 of SEQ ID NO: 4 or SEQ ID NO: 2 are independently selected from 2-Aminobutyric acid (Abu), G, S, A, V, T, I, L, or Allylglycine (AllylGly).

7. The formulation for the use of any one of claims 1 to 3, wherein the peptide has the amino acid sequence LRRFSTAPFAFIDINDVINF (SEQ ID NO: 3).

8. The formulation for the use of claim 1, wherein the patient has diabetic macular edema.

9. The formulation for the use of claim 1, wherein the patient has wet age-related macular degeneration (AMD).

10. The formulation for the use of any one of claims 8 to 9, wherein the formulation is administered after unsuccessful VEGF blockade or inhibitor therapy.

11. The formulation for the use of any one of claims 8 to 9, wherein the condition is refractory or only partially-responsive to VEGF blockade or inhibitor therapy.

12. The formulation for the use of any one of claims 8 to 9, wherein the formulation is administered as an alternative to VEGF blockade or inhibitor therapy.

13. The formulation for the use of any one of claims 8 to 9, wherein the formulation is administered in combination with VEGF blockade therapy.

## Patentansprüche

1. Eine Formulierung, die ein Peptid umfasst, wobei das Peptid eine Aminosäuresequenz mit der Formel LRRFSTXPXXXXDINDVXNF (SEQ ID NO: 4) oder LRRFSTXPXXXXNINNVXNF (SEQ ID NO: 2) umfasst, wobei X eine Standardaminosäure oder eine nicht genetisch kodierte Aminosäure ist,
wobei die Formulierung von 100 µg bis etwa 1000 µg des Peptidmittels umfasst, wobei die Formulierung keine Verkapselung in Partikel beinhaltet,
zur Verwendung bei der Behandlung des diabetischen Makulaödems, des Netzhautvenenverschlusses, der feuchten altersbedingten Makuladegeneration (feuchte AMD) oder der diabetischen Retinopathie, durch intravitreale Injektion wobei die Häufigkeit der Verabreichung nicht häufiger als etwa einmal alle drei Monate ist.

2. Die Formulierung für die Verwendung nach Anspruch 1, wobei die Häufigkeit der Verabreichung einmal alle vier Monate ist.

3. Die Formulierung zur Verwendung nach Anspruch 1, wobei die Häufigkeit der Verabreichung einmal alle sechs Monate ist.

4. Die Formulierung zur Verwendung nach Anspruch 1, wobei die Dosis des Peptids von etwa 200 µg bis etwa 800 µg beträgt.

5. Die Formulierung zur Verwendung nach Anspruch 1, wobei die Dosis des Peptids etwa 400 µg bis etwa 800 µg beträgt.

6. Die Formulierung zur Verwendung nach einem der Ansprüche 1 bis 3, wobei das X an Position 7 von SEQ ID NO: 4 oder SEQ ID NO: 2 M, A oder G ist; X an Position 9 von SEQ ID NO: 4 oder SEQ ID NO: 2 F, A, Y oder G ist; X an Position 10 von SEQ ID NO: 4 oder SEQ ID NO: 2 ist M, A, G, D-Alanin (dA) oder Norleucin (Nle); X an Position 11 von SEQ ID NO: 4 oder SEQ ID NO: 2 ist F, A, Y, G oder 4-Chlorphenylalanin (4-ClPhe); X an Position 12 und Position 18 von SEQ ID NO: 4 oder SEQ ID NO: 2 unabhängig voneinander ausgewählt sind aus 2-Aminobuttersäure (Abu), G, S, A, V, T, I, L oder Allylglycin (AllylGly).

7. Die Formulierung für die Verwendung nach einem der Ansprüche 1 bis 3, wobei das Peptid die Aminosäuresequenz LRRFSTAPFAFIDINDVINF (SEQ ID NO: 3) aufweist.

8. Die Formulierung zur Verwendung nach Anspruch 1, wobei der Patient ein diabetisches Makulaödem hat.

9. Die Formulierung für die Verwendung nach Anspruch 1, wobei der Patient an feuchter altersbedingter Makuladegeneration (AMD) leidet.

10. Formulierung zur Verwendung nach einem der Ansprüche 8 bis 9, wobei die Formulierung nach erfolgloser VEGF-Blockade oder Inhibitortherapie verabreicht wird.

11. Die Formulierung zur Verwendung nach einem der Ansprüche 8 bis 9, wobei der Zustand refraktär ist oder nur teilweise auf die VEGF-Blockade oder Inhibitortherapie anspricht.

12. Die Formulierung zur Verwendung nach einem der Ansprüche 8 bis 9, wobei die Formulierung als Alternative zur VEGF-Blockade oder Inhibitor-Therapie verabreicht wird.

13. Die Formulierung zur Verwendung nach einem der Ansprüche 8 bis 9, wobei die Formulierung in Kombination mit einer VEGF-Blockade-Therapie verabreicht wird.

## Revendications

1. Formulation comprenant un peptide, dans laquelle le peptide comprend une séquence d'acides aminés avec la formule LRRFSTXPXXXXDINDVXNF (SEQ ID NO : 4) ou LRRFSTXPXXXXNINNVXNF (SEQ ID NO : 2), où X est un acide aminé standard ou un acide aminé non génétiquement codé,
dans laquelle la formulation comprend de 100 µg à environ 1000 µg de l'agent peptidique,
dans laquelle la formulation n'implique pas l'encapsulation dans des particules,
pour son utilisation dans le traitement d'un œdème maculaire diabétique, d'une occlusion de la veine rétinienne, d'une dégénérescence maculaire liée à l'âge exsudative (DMLA exsudative), ou d'une rétinopathie diabétique, par injection intravitréenne
dans laquelle la fréquence d'administration n'est pas supérieure à environ une fois tous les trois mois.

2. Formulation pour son utilisation selon la revendication 1, dans laquelle la fréquence d'administration est d'une fois tous les quatre mois.

3. Formulation pour son utilisation selon la revendication 1, dans laquelle la fréquence d'administration est d'une fois tous les six mois.

4. Formulation pour son utilisation selon la revendication 1, dans laquelle la dose du peptide est d'environ 200 µg à environ 800 µg.

5. Formulation pour son utilisation selon la revendication 1, dans laquelle la dose du peptide est d'environ 400 µg à environ 800 µg.

6. Formulation pour son utilisation selon l'une quelconque des revendications 1 à 3, dans laquelle le X en position 7 de SEQ ID NO : 4 ou SEQ ID NO : 2 est M, A, ou G ; X en position 9 de SEQ ID NO : 4 ou SEQ ID NO : 2 est F, A, Y ou G ; X en position 10 de SEQ ID NO : 4 ou SEQ ID NO : 2 est M, A, G, une D-alanine (dA), ou une norleucine (Nle) ; X en position 11 de SEQ ID NO : 4 ou SEQ ID NO : 2 est F, A, Y, G, ou une 4-chlorophénylalanine (4-CIPhe) ; X en position 12 et en position 18 de SEQ ID NO: 4 ou SEQ ID NO: 2 sont indépendamment sélectionnés parmi un acide 2-aminobutyrique (Abu), G, S, A, V, T, I, L, ou une allylglycine (AllylGly).

7. Formulation pour son utilisation selon l'une quelconque des revendications 1 à 3, dans laquelle le peptide a la séquence d'acides aminés LRRFSTAPFAFIDINDVINF (SEQ ID NO : 3).

8. Formulation pour son utilisation selon la revendication 1, dans laquelle le patient présente un œdème maculaire diabétique.

9. Formulation pour son utilisation selon la revendication 1, dans laquelle le patient présente une dégénérescence maculaire liée à l'âge (DMLA) exsudative.

10. Formulation pour son utilisation selon l'une quelconque des revendications 8 à 9, dans laquelle la formulation est administrée après un échec d'une thérapie d'inhibition ou de blocage du VEGF.

11. Formulation pour son utilisation selon l'une quelconque des revendications 8 à 9, dans laquelle l'affection est réfractaire ou ne répond que partiellement à une thérapie d'inhibition ou de blocage du VEGF.

12. Formulation pour son utilisation selon l'une quelconque des revendications 8 à 9, dans laquelle la formulation est administrée comme une variante à une thérapie de blocage ou d'inhibition du VEGF.

13. Formulation pour son utilisation selon l'une quelconque des revendications 8 à 9, dans laquelle la formulation est administrée en combinaison avec une thérapie de blocage du VEGF.


### SEQUENCE LISTING

<110> AsclepiX Therapeutics, LLC The John Hopkins University
<120> COMPOUNDS AND METHODS FOR ACTIVATING TIE2 SIGNALING
<130> ASX-002PC
<150> US 62/403,786
   <151> 2016-10-04
<160> 36
<170> PatentIn version 3.5
<210> 1
   <211> 20
   <212> PRT
   <213> Artificial Sequence
<220>
   <223> Synthetic Polypeptide
<400> 1
   Leu Arg Arg Phe Ser Thr Ala Pro Phe Ala Phe Ile Asp Ile Asn Asp
   1               5                   10                  15
   Val Ile Asn Phe
               20
<210> 2
   <211> 20
   <212> PRT
   <213> Artificial Sequence
<220>
   <223> Synthetic Polypeptide
<220>
   <221> MISC_FEATURE
   <222> (7)..(7)
   <223> M, A, or G
<220>
   <221> MISC_FEATURE
   <222> (9)..(9)
   <223> F, A, Y, or G
<220>
   <221> MISC_FEATURE
   <222> (10)..(10)
   <223> M, A, G, D-Alanine, or norleucine
<220>
   <221> MISC_FEATURE
   <222> (11)..(11)
   <223> F, A, Y, G, or 4-chlorophenylalanine
<220>
   <221> MISC_FEATURE
   <222> (12)..(12)
   <223> Aminobutyric acid, G, S, A, V, T, I, L, or Allylglycine
<220>
   <221> MISC_FEATURE
   <222> (18)..(18)
   <223> Aminobutyric acid, G, S, A, V, T, I, L, or Allylglycine
<400> 2
   Leu Arg Arg Phe Ser Thr Xaa Pro Xaa Xaa Xaa Xaa Asn Ile Asn Asn
   1               5                   10                  15
   Val Xaa Asn Phe
               20
<210> 3
   <211> 20
   <212> PRT
   <213> Artificial Sequence
<220>
   <223> Synthetic Polypeptide
<400> 3
   Leu Arg Arg Phe Ser Thr Ala Pro Phe Ala Phe Ile Asp Ile Asn Asp
   1               5                   10                  15
   Val Ile Asn Phe
               20
<210> 4
   <211> 20
   <212> PRT
   <213> Artificial Sequence
<220>
   <223> Synthetic Polypeptide
<220>
   <221> MISC_FEATURE
   <222> (7)..(7)
   <223> M, A, or G
<220>
   <221> MISC_FEATURE
   <222> (9)..(9)
   <223> F, A, Y, or G
<220>
   <221> MISC_FEATURE
   <222> (10)..(10)
   <223> M, A, G, D-Alanine, or norleucine
<220>
   <221> MISC_FEATURE
   <222> (11)..(11)
   <223> F, A, Y, G, or 4-chlorophenylalanine
<220>
   <221> MISC_FEATURE
   <222> (12)..(12)
   <223> Aminobutyric acid, G, S, A, V, T, I, L, or Allylglycine
<220>
   <221> MISC_FEATURE
   <222> (18)..(18)
   <223> Aminobutyric acid, G, S, A, V, T, I, L, or Allylglycine
<400> 4
   Leu Arg Arg Phe Ser Thr Xaa Pro Xaa Xaa Xaa Xaa Asp Ile Asn Asp
   1               5                   10                  15
   Val Xaa Asn Phe
               20
<210> 5
   <211> 20
   <212> PRT
   <213> Artificial Sequence
<220>
   <223> Synthetic Polypeptide
<220>
   <221> MISC_FEATURE
   <222> (12)..(12)
   <223> 2-Aminobutyric acid
<220>
   <221> MISC_FEATURE
   <222> (18)..(18)
   <223> 2-Aminobutyric acid
<400> 5
   Leu Arg Arg Phe Ser Thr Met Pro Phe Met Phe Xaa Asn Ile Asn Asn
   1               5                   10                  15
   Val Xaa Asn Phe
               20
<210> 6
   <211> 20
   <212> PRT
   <213> Artificial Sequence
<220>
   <223> Synthetic Polypeptide
<220>
   <221> MISC_FEATURE
   <222> (12)..(12)
   <223> 2-Aminobutyric acid
<220>
   <221> MISC_FEATURE
   <222> (18)..(18)
   <223> 2-Aminobutyric acid
<400> 6
   Leu Arg Arg Phe Ser Thr Met Pro Ala Met Phe Xaa Asn Ile Asn Asn
   1               5                   10                  15
   Val Xaa Asn Phe
               20
<210> 7
   <211> 20
   <212> PRT
   <213> Artificial Sequence
<220>
   <223> Synthetic Polypeptide
<220>
   <221> MISC_FEATURE
   <222> (12)..(12)
   <223> 2-Aminobutyric acid
<220>
   <221> MISC_FEATURE
   <222> (18)..(18)
   <223> 2-Aminobutyric acid
<400> 7
   Leu Arg Arg Phe Ser Thr Met Pro Phe Ala Phe Xaa Asn Ile Asn Asn
   1               5                   10                  15
   Val Xaa Asn Phe
               20
<210> 8
   <211> 20
   <212> PRT
   <213> Artificial Sequence
<220>
   <223> Synthetic Polypeptide
<220>
   <221> MISC_FEATURE
   <222> (12)..(12)
   <223> 2-Aminobutyric acid
<220>
   <221> MISC_FEATURE
   <222> (18)..(18)
   <223> 2-Aminobutyric acid
<400> 8
   Leu Arg Arg Phe Ser Thr Met Pro Phe Met Ala Xaa Asn Ile Asn Asn
   1               5                   10                  15
   Val Xaa Asn Phe
               20
<210> 9
   <211> 20
   <212> PRT
   <213> Artificial Sequence
<220>
   <223> Synthetic Polypeptide
<220>
   <221> MISC_FEATURE
   <222> (10)..(10)
   <223> norleucine
<220>
   <221> MISC_FEATURE
   <222> (12)..(12)
   <223> 2-Aminobutyric acid
<220>
   <221> MISC_FEATURE
   <222> (18)..(18)
   <223> 2-Aminobutyric acid
<400> 9
   Leu Arg Arg Phe Ser Thr Met Pro Phe Xaa Phe Xaa Asn Ile Asn Asn
   1               5                   10                  15
   Val Xaa Asn Phe
               20
<210> 10
   <211> 20
   <212> PRT
   <213> Artificial Sequence
<220>
   <223> Synthetic Polypeptide
<220>
   <221> MISC_FEATURE
   <222> (11)..(11)
   <223> 4-chlorophenylalanine
<220>
   <221> MISC_FEATURE
   <222> (12)..(12)
   <223> 2-Aminobutyric acid
<220>
   <221> MISC_FEATURE
   <222> (18)..(18)
   <223> 2-Aminobutyric acid
<400> 10
   Leu Arg Arg Phe Ser Thr Met Pro Phe Met Xaa Xaa Asn Ile Asn Asn
   1               5                   10                  15
   Val Xaa Asn Phe
               20
<210> 11
   <211> 20
   <212> PRT
   <213> Artificial Sequence
<220>
   <223> Synthetic Polypeptide
<400> 11
   Leu Arg Arg Phe Ser Thr Met Pro Phe Met Phe Ser Asn Ile Asn Asn
   1               5                   10                  15
   Val Ser Asn Phe
               20
<210> 12
   <211> 20
   <212> PRT
   <213> Artificial Sequence
<220>
   <223> Synthetic Polypeptide
<400> 12
   Leu Arg Arg Phe Ser Thr Met Pro Phe Met Phe Ala Asn Ile Asn Asn
   1               5                   10                  15
   Val Ala Asn Phe
               20
<210> 13
   <211> 20
   <212> PRT
   <213> Artificial Sequence
<220>
   <223> Synthetic Polypeptide
<400> 13
   Leu Arg Arg Phe Ser Thr Met Pro Phe Met Phe Ile Asn Ile Asn Asn
   1               5                   10                  15
   Val Ile Asn Phe
               20
<210> 14
   <211> 20
   <212> PRT
   <213> Artificial Sequence
<220>
   <223> Synthetic Polypeptide
<400> 14
   Leu Arg Arg Phe Ser Thr Met Pro Phe Met Phe Thr Asn Ile Asn Asn
   1               5                   10                  15
   Val Thr Asn Phe
               20
<210> 15
   <211> 20
   <212> PRT
   <213> Artificial Sequence
<220>
   <223> Synthetic Polypeptide
<220>
   <221> MISC_FEATURE
   <222> (12)..(12)
   <223> Allylglycine
<220>
   <221> MISC_FEATURE
   <222> (18)..(18)
   <223> Allylglycine
<400> 15
   Leu Arg Arg Phe Ser Thr Met Pro Phe Met Phe Xaa Asn Ile Asn Asn
   1               5                   10                  15
   Val Xaa Asn Phe
               20
<210> 16
   <211> 20
   <212> PRT
   <213> Artificial Sequence
<220>
   <223> Synthetic Polypeptide
<400> 16
   Leu Arg Arg Phe Ser Thr Met Pro Phe Met Phe Val Asn Ile Asn Asn
   1               5                   10                  15
   Val Val Asn Phe
               20
<210> 17
   <211> 20
   <212> PRT
   <213> Artificial Sequence
<220>
   <223> Synthetic Polypeptide
<220>
   <221> MISC_FEATURE
   <222> (10)..(10)
   <223> D-Alanine
<400> 17
   Leu Arg Arg Phe Ser Thr Met Pro Phe Xaa Phe Ile Asn Ile Asn Asn
   1               5                   10                  15
   Val Ile Asn Phe
               20
<210> 18
   <211> 20
   <212> PRT
   <213> Artificial Sequence
<220>
   <223> Synthetic Polypeptide
<400> 18
   Leu Arg Arg Phe Ser Thr Met Pro Phe Ala Phe Ile Asn Ile Asn Asn
   1               5                   10                  15
   Val Ile Asn Phe
               20
<210> 19
   <211> 20
   <212> PRT
   <213> Artificial Sequence
<220>
   <223> Synthetic Polypeptide
<400> 19
   Leu Arg Arg Phe Ser Thr Ala Pro Phe Ala Phe Ile Asn Ile Asn Asn
   1               5                   10                  15
   Val Ile Asn Phe
               20
<210> 20
   <211> 20
   <212> PRT
   <213> Artificial Sequence
<220>
   <223> Synthetic Polypeptide
<220>
   <221> MISC_FEATURE
   <222> (10)..(10)
   <223> D-Alanine
<400> 20
   Leu Arg Arg Phe Ser Thr Ala Pro Phe Xaa Phe Ile Asp Ile Asn Asp
   1               5                   10                  15
   Val Ile Asn Phe
               20
<210> 21
   <211> 20
   <212> PRT
   <213> Artificial Sequence
<220>
   <223> Synthetic Polypeptide
<400> 21
   Leu Arg Arg Phe Ser Thr Ala Pro Phe Ala Phe Ile Asp Ile Asn Asp
   1               5                   10                  15
   Val Ile Asn Trp
               20
<210> 22
   <211> 23
   <212> PRT
   <213> Artificial Sequence
<220>
   <223> Synthetic Polypeptide
<220>
   <221> MISC_FEATURE
   <222> (1)..(1)
   <223> D-Leucine
<220>
   <221> MISC_FEATURE
   <222> (4)..(4)
   <223> D-Leucine
<400> 22
   Xaa Arg Arg Xaa Arg Arg Phe Ser Thr Ala Pro Phe Ala Phe Ile Asp
   1               5                   10                  15
   Ile Asn Asp Val Ile Asn Phe
               20
<210> 23
   <211> 20
   <212> PRT
   <213> Artificial Sequence
<220>
   <223> Synthetic Polypeptide
<220>
   <221> MISC_FEATURE
   <222> (20)..(20)
   <223> D-Phenylalanine
<400> 23
   Leu Arg Arg Phe Ser Thr Ala Pro Phe Ala Phe Ile Asp Ile Asn Asp
   1               5                   10                  15
   Val Ile Asn Xaa
               20
<210> 24
   <211> 20
   <212> PRT
   <213> Artificial Sequence
<220>
   <223> Synthetic Polypeptide
<220>
   <221> MISC_FEATURE
   <222> (1)..(1)
   <223> D-Leucine
<220>
   <221> MISC_FEATURE
   <222> (20)..(20)
   <223> D-Phenylalanine
<400> 24
   Xaa Arg Arg Phe Ser Thr Ala Pro Phe Ala Phe Ile Asp Ile Asn Asp
   1 5 10 15
   Val Ile Asn Xaa
   20
<210> 25
   <211> 9
   <212> PRT
   <213> Artificial Sequence
<220>
   <223> Synthetic Polypeptide
<220>
   <221> MISC_FEATURE
   <222> (2)..(2)
   <223> 2-Aminobutyric acid
<220>
   <221> MISC_FEATURE
   <222> (8)..(8)
   <223> 2-Aminobutyric acid
<400> 25
   Phe Xaa Asn Ile Asn Asn Val Xaa Asn
   1 5
<210> 26
   <211> 9
   <212> PRT
   <213> Artificial Sequence
<220>
   <223> Synthetic Polypeptide
<400> 26
   Phe Thr Asn Ile Asn Asn Val Thr Asn
   1 5
<210> 27
   <211> 10
   <212> PRT
   <213> Artificial Sequence
<220>
   <223> Synthetic Polypeptide
<400> 27
   Phe Ile Asn Ile Asn Asn Val Ile Asn Phe
   1 5 10
<210> 28
   <211> 10
   <212> **PRT**
   <213> Artificial Sequence
<220>
   <223> Synthetic Polypeptide
<400> 28
   Phe Ser Asn Ile Asn Asn Val Ser Asn Phe
   1               5                   10
<210> 29
   <211> 10
   <212> PRT
   <213> Artificial Sequence
<220>
   <223> Synthetic Polypeptide
<400> 29
   Phe Ala Asn Ile Asn Asn Val Ala Asn Phe
   1               5                   10
<210> 30
   <211> 10
   <212> PRT
   <213> Artificial Sequence
<220>
   <223> Synthetic Polypeptide
<220>
   <221> MISC_FEATURE
   <222> (2)..(2)
   <223> Allylglycine
<220>
   <221> MISC_FEATURE
   <222> (8)..(8)
   <223> Allylglycine
<400> 30
   Phe Xaa Asn Ile Asn Asn Val Xaa Asn Phe
   1               5                   10
<210> 31
   <211> 10
   <212> PRT
   <213> Artificial Sequence
<220>
   <223> Synthetic Polypeptide
<400> 31
   Phe Val Asn Ile Asn Asn Val Val Asn Phe
   1               5                   10
<210> 32
   <211> 10
   <212> **PRT**
   <213> **Artificial** Sequence
<220>
   <223> Synthetic Polypeptide
<400> 32
   Phe Ile Asp Ile Asn Asp Val Ile Asn Phe
   1               5                   10
<210> 33
   <211> 10
   <212> **PRT**
   <213> **Artificial** Sequence
<220>
   <223> Synthetic Polypeptide
<400> 33
   Phe Ile Asp Ile Asn Asp Val Ile Asn Trp
   1               5                   10
<210> 34
   <211> 9
   <212> **PRT**
   <213> **Artificial** Sequence
<220>
   <223> Synthetic Polypeptide
<400> 34
   Phe Thr Asp Ile Asn Asp Val Thr Asn
   1               5
<210> 35
   <211> 10
   <212> PRT
   <213> Artificial Sequence
<220>
   <223> Synthetic Polypeptide
<220>
   <221> MISC_FEATURE
   <222> (2)..(2)
   <223> 2-Aminobutyric acid
<220>
   <221> MISC_FEATURE
   <222> (8)..(8)
   <223> 2-Aminobutyric acid
<400> 35
   Ala Xaa Asn Ile Asn Asn Val Xaa Asn Phe
   1               5                   10
<210> 36
   <211> 10
   <212> PRT
   <213> Artificial Sequence
<220>
   <223> Synthetic Polypeptide
<220>
   <221> MISC_FEATURE
   <222> (1)..(1)
   <223> 4-chlorophenylalanine
<220>
   <221> MISC_FEATURE
   <222> (2)..(2)
   <223> 2-Aminobutyric acid
<220>
   <221> MISC_FEATURE
   <222> (8)..(8)
   <223> 2-Aminobutyric acid
<400> 36
   Xaa Xaa Asn Ile Asn Asn Val Xaa Asn Phe
   1               5                   10